(19) Europäisches Patentamt
European Patent Office
Office européen des brevets

(11) **EP 2 654 941 B1**

(12) **EUROPÄISCHE PATENTSCHRIFT**

(45) Veröffentlichungstag und Bekanntmachung des Hinweises auf die Patenterteilung:
**26.11.2014  Patentblatt 2014/48**

(21) Anmeldenummer: **11802692.1**

(22) Anmeldetag: **13.12.2011**

(51) Int Cl.:
**B01J 19/24** *(2006.01)*        **C07C 5/48** *(2006.01)*

(86) Internationale Anmeldenummer:
**PCT/EP2011/072636**

(87) Internationale Veröffentlichungsnummer:
**WO 2012/084609 (28.06.2012 Gazette 2012/26)**

(54) **REAKTOR ZUR DURCHFÜHRUNG EINER AUTOTHERMEN GASPHASENDEHYDRIERUNG**

REACTOR FOR CARRYING OUT AN AUTOTHERMAL GAS- PHASE DEHYDRATION

RÉACTEUR POUR LA MISE EN ŒUVRE D'UNE DÉSHYDRATATION AUTOTHERMIQUE EN PHASE GAZEUSE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **21.12.2010  EP 10196216**
                **01.06.2011  EP 11168408**
                **22.11.2011  EP 11190117**

(43) Veröffentlichungstag der Anmeldung:
**30.10.2013  Patentblatt 2013/44**

(73) Patentinhaber: **BASF SE**
**67056 Ludwigshafen (DE)**

(72) Erfinder:
• **OLBERT, Gerhard**
**69221 Dossenheim (DE)**

• **WEGERLE, Ulrike**
**67550 Worms (DE)**
• **KOLIOS, Grigorios**
**67435 Neustadt (DE)**
• **KOSTOVA, Albena**
**68163 Mannheim (DE)**
• **KESSEL, Jasmina**
**68165 Mannheim (DE)**
• **WECK, Alexander**
**67251 Freinsheim (DE)**
• **REZAI, Alireza**
**68161 Mannheim (DE)**

(56) Entgegenhaltungen:
**WO-A1-2011/067235        US-A1- 2008 119 673**
**US-A1- 2009 292 030**

**Beschreibung**

Die Erfindung betrifft einen Reaktor zur Durchführung von autothermen Gasphasendehydrierungen unter Verwendung eines heterogenen Katalysators, der als Monolith ausgebildet ist sowie ein Verfahren unter Verwendung des Reaktors.

Keramische oder metallische Monolithe sind als Katalysatorträger für Edelmetallkatalysatoren in der mobilen und stationären Abgasreinigung etabliert. Die Kanäle bieten der Strömung einen geringen Strömungswiderstand bei gleichzeitig gleichmäßiger Zugänglichkeit der äußeren Katalysatoroberfläche für gasförmige Reaktionsmedien. Dies ist vorteilhaft gegenüber regellosen Haufwerken, bei denen durch unzählige Umlenkungen bei der Strömung um die Partikel ein großer Druckverlust entsteht und die Katalysatoroberfläche eventuell nicht gleichmäßig genutzt wird. Der Einsatz von Monolithen ist generell interessant für katalytische Prozesse mit hohen Volumenströmen und adiabater Reaktionsführung bei hohen Temperaturen. Diese Merkmale treffen in der chemischen Produktionstechnik insbesondere für Dehydrierungsreaktionen zu, die in einem Temperaturbereich von 400 °C bis zu 700 °C ablaufen.

Fortschritte in der Katalysatortechnik ermöglichen die selektive Verbrennung des Dehydrierwasserstoffes in Anwesenheit von Kohlenwasserstoffen, wie beispielsweise in US 7,034,195 beschrieben. Eine derartige Fahrweise wird als autotherme Dehydrierung bezeichnet und erlaubt, Dehydrierreaktoren direkt zu beheizen, so dass aufwändige Vorrichtungen zur indirekten Vor- und Zwischenheizung des Reaktionsgemisches entfallen. Ein derartiges Verfahren ist beispielsweise in US 2008/0119673 beschrieben. Dieses Verfahren besitzt jedoch den gravierenden Nachteil, dass die Dehydrierung an einem heterogenen Katalysator in Pelletform durchgeführt wird: Der hohe Strömungswiderstand von Pelletschüttungen erfordert einen großen Reaktorquerschnitt und eine entsprechend niedrige Durchströmungsgeschwindigkeit, um den Druckabfall in der katalytisch aktiven Schicht zu begrenzen. Dieser Nachteil wird durch eine sehr aufwändige Vorrichtung zur Dosierung und Verteilung des Sauerstoffes ausgeglichen, was den Vorteil der autothermen Dehydrierung beeinträchtigt.

Aus der nicht vorveröffentlichten europäischen Patentanmeldung EP 09177649.2 ist ein Reaktor sowie ein Verfahren zur autothermen Gasphasendehydrierung von Kohlenwasserstoffen unter Einsatz von als Monolithe ausgebildeten heterogenen Katalysatoren bekannt, das eine Beherrschung der brennbaren Reaktionsmedien bei den hohen Reaktionstemperaturen, häufig im Bereich von etwa 400 bis 700 °C, sowie eine einfache Zugänglichkeit und Handhabung der Monolithe, insbesondere beim Rüsten des Reaktors sowie bei einem Katalysatorwechsel, gewährleistet.

Die EP 09177649.2 stellt einen Reaktor in Form eines liegenden Zylinders zur Durchführung einer autothermen Gasphasendehydrierung eines kohlenwasserstoffhaltigen Gasstromes mit einem Sauerstoff enthaltenden Gasstrom unter Erhalt eines Reaktionsgasgemisches an einem heterogenen Katalysator, der als Monolith ausgebildet ist, zur Verfügung, wobei

- der Innenraum des Reaktors durch ein lösbar, in Längsrichtung des Reaktors angeordnetes kreiszylindrisches oder prismatisches, in Umfangsrichtung gasdichtes, an beiden Stirnseiten desselben offenes Gehäuse G in

- einen Innenbereich A, mit einer oder mehreren katalytisch aktiven Zonen, worin jeweils eine Packung aus aufeinander, nebeneinander und hintereinander gestapelten Monolithen und vor jeder katalytisch aktiven Zone jeweils eine Mischzone mit festen Einbauten vorgesehen ist und

- einen koaxial zum Innenbereich A angeordneten Außenbereich B aufgeteilt ist,

- mit einer oder mehreren Zuführleitungen für den zu dehydrierenden kohlenwasserstoffhaltigen Gasstrom in den Außenbereich B, Umlenkung des zu dehydrierenden Kohlenwasserstoffstroms an einem Ende des Reaktors und Zuführung über einen Strömungsgleichrichter in den Innenbereich A,

- mit einer oder mehreren, unabhängig voneinander regelbaren Zuführleitungen, wobei jede Zuführleitung eine oder mehrere Verteilerkammern versorgt, für den Sauerstoff enthaltenden Gasstrom in jede der Mischzonen sowie

- mit einer Abführleitung für das Reaktionsgemisch der autothermen Gasphasendehydrierung am Ende des Reaktors wie die Zuführleitung für den zu dehydrierenden Kohlenwasserstoffstrom.

An dem Reaktorende, an dem die Abführleitung für das Reaktionsgasgemisch der autothermen Gasphasendehydrierung angeordnet ist, ist vorteilhaft ein Rohrbündelwärmetauscher vorgesehen, mit einem Bündel von Rohren, durch die das Reaktionsgasgemisch zur autothermen Gasphasendehydrierung geleitet wird, sowie mit Zwischenräumen zwischen den Rohren, durch die, im Gegenstrom zum Reaktionsgemisch der autothermen Gasphasendehydrierung, der zu dehydrierende kohlenwasserstoffhaltige Gasstrom geleitet wird.

Der obige Reaktor weist jedoch aus sicherheitstechnischer Sicht Nachteile auf, weil die Sauerstoff führenden Leitungen durch den Raum führen, in dem der kohlenwasserstoffhaltige Strom zirkuliert. Bei einer Leckage durch einen Rohrriss kann es daher zur Entzündung/Explosion kommen. Darüber hinaus ist in der Ausführungsform mit Rohrbündelwärmetauscher, der aus dem Reaktormantel herausragt, die Abdichtung desselben nur unbefriedigend gelöst.

Es war daher Aufgabe der Erfindung, einen verbesserten Reaktor zur Verfügung zu stellen, der die obigen Nachteile überwindet.

Die Aufgabe wird gelöst durch einen Reaktor in Form eines liegenden Zylinders oder Prismas zur Durchführung einer autothermen Gasphasendehydrierung eines kohlenwasserstoffhaltigen Gasstromes mit einem Sauerstoff enthaltenden Gasstrom unter Erhalt eines Reaktionsgasgemisches, an einem heterogegen Katalysator, der als Monolith ausgebildet ist, wobei

- der Innenraum des Reaktors durch ein lösbar, in Längsrichtung des Reaktors angeordnetes kreiszylindrisches oder prismatisches, gasdichtes Gehäuse G in

- einen Innenbereich A, mit einer oder mehreren katalytisch aktiven Zonen, worin jeweils eine Packung aus aufeinander, nebeneinander und hintereinander gestapelten Monolithen und vor jeder katalytisch aktiven Zone jeweils eine Mischzone mit festen Einbauten vorgesehen ist und

- einen koaxial zum Innenbereich A angeordneten Außenbereich B aufgeteilt ist, und wobei

  - an einem Reaktorende im Anschluss an das Gehäuse G ein Wärmetauscher vorgesehen ist

- mit einer oder mehreren Zuführleitungen für den zu dehydrierenden kohlenwasserstoffhaltigen Gasstrom,

  - mit einer oder mehreren, unabhängig voneinander regelbaren Zuführleitungen, wobei jede Zuführleitung eine oder mehrere Verteilerkammern versorgt, für den Sauerstoff enthaltenden Gasstrom in jede der Mischzonen sowie

  - mit einer Abführleitung für das Reaktionsgasgemisch der autothermen Gasphasendehydrierung,

der dadurch gekennzeichnet ist, dass der Außenbereich B mit einem unter den Reaktionsbedingungen der autothermen Gasphasendehydrierung inerten Gas beaufschlagbar ist und dass der zu dehydrierende kohlenwasserstoffhaltige Gasstrom über eine Zuführleitung in den Wärmetauscher eingeleitet, durch das Reaktionsgasgemisch im Gegenstrom durch indirekten Wärmetausch aufgeheizt und weiter an das dem Wärmetauscher entgegengesetzte Ende des Reaktors geleitet, dort umgelenkt, über einen Strömungsgleichrichter in den Innenbereich A eingeleitet, und in den Mischzonen mit dem Sauerstoff enthaltenden Gasstrom vermischt wird, worauf im Innenbereich A des Reaktors die autotherme Gasphasendehydrierung stattfindet, und dass im Innenbereich A zwei oder mehrere katalytisch aktive Zonen mit jeweils einer Packung aus aufeinander, nebeneinander und hintereinander gestapelten Monolithen vorgesehen sind.

Erfindungsgemäß wird somit ein Reaktor mit einem Reaktormantel, d. h. einer drucktragenden Hülle, vorgeschlagen, die nicht mediumberührt ist, weder durch den kohlenwasserstoffhaltigen noch durch den sauerstoffhaltigen Strom.

In Längsrichtung des Reaktors ist ein zylindrisches oder prismatisches Gehäuse G angeordnet, das den Innenraum des Reaktors in einen Innenbereich A und einen konzentrisch um denselben angeordneten Außenbereich B aufteilt.

Der Außenbereich B ist mit einem unter den Reaktionsbedingungen der autothermen Gasphasendehydrierung inerten Gas beaufschlagt, d.h. einem Gas oder Gasgemisch, das an der Reaktion der autothermen Gasphasendehydrierung nicht unmittelbar beteiligt ist, insbesondere ausgewählt aus Wasser, Kohlendioxid, Stickstoff und Edelgasen oder Gemischen hiervon. Bevorzugt wird als unter den Reaktionsbedingungen der autothermen Gasphasendehydrierung inertes Gas Wasserdampf eingesetzt, da dieser in einfacher Weise, durch Auskondensieren, aus dem Reaktionsgasgemisch wieder abgetrennt werden kann.

Bevorzugt wird das unter den Reaktionsbedingungen der autothermen Gasphasendehydrierung inerte Gas als Purge-Gasstrom mit einem geringen Massenstrom im Vergleich zum Massenstrom des kohlenwasserstoffhaltigen Gasstromes, d.h. einem Massenstrom von 1/5 bis 1/100, bevorzugt einem Massenstrom von 1/10 bis 1/50, bezogen auf den Massenstrom des kohlenwasserstoffhaltigen Gasstromes, unter geringem Überdruck von 2 bis 50 mbar, bevorzugt von 25 bis 30 mbar, bezogen auf den Druck im Innenbereich A durch denselben geleitet.

Der Purge-Gasstrom kann vorteilhaft durch den Außenbereich B geleitet werden, indem er an einem Reaktorende über eine oder mehrere Zuführleitungen in den Außenbereich B des Reaktors eingeleitet und am entgegengesetzten Reaktorende in den Innenbereich A des Reaktors weitergeleitet wird, bevorzugt über eine oder mehrere, vorteilhaft in einem Winkel verschieden von 90° zur Zuführleitung, für den zu dehydrierenden kohlenwasserstoffhaltigen Gasstrom angeordnete Verbindungsleitung(en).

Die eine oder mehrere Verbindungsleitung(en), die den Purge-Gasstrom aus dem Außenbereich B in den Innenbereich A leiten, sind bevorzugt rückströmungsfrei ausgebildet, beispielsweise durch eine gewendelte Form derselben. Der Einlass aus dem Außenbereich B in die Verbindungsleitung für den Purge-Gasstrom, soll bevorzugt so hoch wie möglich im Außenbereich B des Reaktors angeordnet sein.

Der Purge-Gasstrom spült permanent den Außenbereich B des Reaktors und hält diesen frei von Komponenten des Reaktionsgasgemischs.

An einem Ende des Gehäuses G ist ein Wärmetauscher angeschlossen, der insbesondere ein Rohrbündelwärmetauscher oder ein Plattenwärmetauscher sein kann. Für den Fall des Rohrbündelwärmetauschers ist der Anschluss zwischen demselben und dem Gehäuse G so ausgebildet, dass der Innenbereich A mit dem Innenraum der Rohre des Rohrbündelwärmetauschers kommuniziert. Für den Fall eines Plattenwärmetauschers kommuniziert der Innenbereich A des Reaktors mit den Spalten zwischen den Platten des Plattenwärmetauschers.

Der Zwischenraum zwischen den Rohren des Rohrbündelwärmetauschers bzw. zwischen jeweils zwei zu einer Wärmetauscherplatte zusammengeschweißten Blechen des Plattenwärmetauschers ist über eine Leitung, die an das dem Wärmetauscher entgegengesetzte Ende des Reaktors führt und dort umgelenkt wird, mit dem dem Wärmetauscher entgegengesetzten Ende des Gehäuses G und somit dem Innenbereich des Reaktors gasdicht gegenüber dem Außenbereich B verbunden.

Der kohlenwasserstoffhaltige Strom wird durch den Zwischenraum zwischen den Rohren des Rohrbündelwärmetauschers bzw., für den Fall eines Plattenwärmetauschers, durch die Zwischenräume zwischen den jeweils eine Wärmetauscherplatte bildenden Blechen geleitet, mit dem im Gegenstrom durch die Rohre bzw. durch die Spalte zwischen den Platten des Plattenwärmetauschers zirkulierenden Produktgasstrom aufgeheizt, an das entgegengesetzte Ende des Reaktors geführt, dort umgelenkt und in den Innenbereich A des Gehäuses eingeleitet.

Die autotherme Gasphasendehydrierung findet an einem heterogenen Katalysator statt, der in Form von Monolithen vorliegt.

Als Monolith wird vorliegend ein einstückiger, parallelepipedischer Block mit einer Vielzahl von parallel zueinander angeordneten, durchgehenden Kanälen mit engem Querschnitt, im Bereich von etwa 0,5 bis 4 mm, verstanden.

Die Monolithe sind bevorzugt aus einem keramischen Werkstoff als Trägermaterial gebildet, worauf eine katalytisch aktive Schicht, bevorzugt nach dem sogenannten Wash-Coating-Verfahren, aufgebracht ist.

Das gängigste Material für monolithische Strukturen ist Cordierit (ein Keramikmaterial, das aus Magnesiumoxid, Siliciumoxid und Aluminiumoxid im Verhältnis 2:5:2 besteht). Andere Materialien, deren Monolithstrukturen im Handel erhältlich sind, sind Metalle, Mullit (Mischoxid von Siliciumoxid und Aluminiumoxid, Verhältnis 2:3) und Siliciumcarbid. Diese Materialien haben ähnlich wie Cordierit eine niedrige spezifische BET-Oberfläche (BET = Brunauer, Emmet und Teller) (z.B. für Cordierit typischerweise 0,7 m$^2$/g).

Monolithische Keramikelemente sind mit Zelldichten von 25 - 1600 cpsi (Zellen pro Quadratzoll, entspricht einer Zellgröße von 5 - 0,6 mm) erhältlich. Durch Verwendung einer höheren Zelldichte nimmt die geometrische Oberfläche zu, so dass der Katalysator effizienter verwendet werden kann. Nachteile von höheren Zelldichten sind ein etwas schwierigeres Herstellungsverfahren, eine schwierigere Washcoat-Beschichtung und ein höherer Druckverlust über den Reaktor. Der Druckverlust bleibt jedoch für Monolithen mit hoher Zelldichte im Vergleich zu einem Füllkörperreaktor sehr gering (in der Regel um einen Faktor 10 geringer), was auf die geraden Monolithkanäle zurückzuführen ist.

Zur Herstellung von monolithischen Keramikelementen kann man eine Mischung von Talk, Ton und einer aluminiumoxidliefernden Komponenten und Siliciumoxid herstellen, die Mischung zur Bildung einer Formmasse mischen, die Mischung formen, die Rohware trocknen und sie bei einer Temperatur von 1200 bis 1500 °C erhitzen, wobei man eine Keramik erhält, die hauptsächlich Cordierit enthält und einen niedrigen Wärmeausdehnungskoeffizienten aufweist. Allgemein gesprochen kann man eine Paste mit entsprechenden rheologischen Eigenschaften und entsprechender rheologischer Zusammensetzung zu einem Monolithträger extrudieren. Die Paste besteht in der Regel aus einer Mischung von Keramikpulvern geeigneter Größe, anorganischen und/oder organischen Additiven, Lösungsmittel (Wasser), Peptisierungsmittel (Säure) zur Einstellung des pH-Werts und einem permanenten Bindemittel (kolloidale Lösung oder Sol). Bei den Additiven kann es sich um einen Weichmacher oder ein Tensid zur Einstellung der Viskosität der Paste oder ein temporäres Bindemittel, das später abgebrannt werden kann, handeln. Zuweilen werden Glas- oder Kohlefasern zur Erhöhung der mechanischen Festigkeit des Monolithen zugesetzt. Das permanente Bindemittel sollte die innere Festigkeit des Monolithen verbessern.

Cordierit-Monolithe können aus einer Charge hergestellt werden, die aus Talk, Kaolin, calciniertem Kaolin und Aluminiumoxid besteht und zusammen eine chemische Verbindung aus 45 bis 55 Gew.-% SiO$_2$, 32 bis 40 Gew.-% Al$_2$O$_3$ und 12 bis 15 Gew.-% MgO liefern. Talk ist ein Material, das hauptsächlich aus Magnesiumsilicathydrat, Mg$_3$Si$_4$O$_{10}$(OH)$_2$ besteht. Der Talk kann je nach Quelle und Reinheit auch mit anderen Mineralien wie Tremolit (CaMg$_3$(SiO$_3$)$_4$), Serpentin (3MgO.2SiO$_2$, 2H$_2$O), Anthophyllit (Mg$_7$(OH)$_2$(Si$_4$O$_{11}$)$_2$), Magnesit (MgCO$_3$), Glimmer und Chlorit vergesellschaftet sein.

Durch Extrusion können auch Monolithe aus anderen Materialien wie SiC, B$_4$C, Si$_3$N$_4$, BN, AlN, Al$_2$O$_3$, ZrO$_2$,

Mullit, Al-Titanat, $ZrB_2$, Sialon, Perowskit, Kohlenstoff und $TiO_2$ hergestellt werden.

Von Bedeutung hinsichtlich der Eigenschaften der Monolithprodukte sind bei der Extrusion neben der Qualität der Düse, der Art und den Eigenschaften der zur Herstellung der formbaren Mischung verwendeten Materialien auch die zugesetzten Additive, der pH-Wert, der Wassergehalt und die bei der Extrusion verwendete Kraft. Bei den bei der Extrusion angewandten Additiven handelt es sich beispielsweise um Cellulosen, $CaCl_2$, Ethylenglykole, Diethylenglykole, Alkohole, Wachs, Paraffin, Säuren und hitzebeständige anorganische Fasern. Neben Wasser können auch andere Lösungsmittel verwendet werden, wie Ketone, Alkohole und Ether. Der Zusatz von Additiven kann zu verbesserten Eigenschaften der Monolithe, wie der Bildung von Mikrorissen, die die Temperaturwechselbeständigkeit verbessert, besserer Porosität und besserem Absorptionsvermögen und erhöhter mechanischer Festigkeit oder geringer Wärmeausdehnung führen.

Die nackte monolithische Struktur wird mit einer Katalysatorträgerschicht, die ein oder mehrere keramischen Oxide umfasst, oder einer Katalysatorschicht, die die katalytisch wirksamen Metalle und die fakultativen weiteren (Promotor-)Elemente bereits auf dem keramischen Oxidträgermaterial geträgert umfasst, beschichtet, wobei die Beschichtung nach einer Washcoat-Beschichtungsmethode hergestellt wird.

Die makroporöse Struktur von Keramikmonolithen erleichtert die Verankerung der Washcoatschicht. Die Art und Weise der Washcoat-Beschichtung kann in zwei Methoden unterteilt werden: Man kann den makroporösen Träger (teilweise) mit dem eine große Oberfläche aufweisenden Washcoatmaterial füllen oder einen Washcoat als Schicht in den Poren des Keramikträgers abscheiden. Das Porenfüllen führt zur stärksten Wechselwirkung zwischen Monolith und Washcoat, da der größte Teil der Washcoatschicht tatsächlich in den Poren des Trägers fixiert ist und nicht nur an die äußere Oberfläche der Monolithkanäle gebunden ist. Diese Art von Beschichtung wird mit einer Lösung (oder einem Sol) des abzuscheidenden Materials oder mit einer sehr kleine kolloidale Teilchen enthaltenden Lösung durchgeführt. Der Nachteil des Beschichtens mittels Porenfüllung besteht darin, dass die abscheidbare Beschichtungsmenge begrenzt ist, da die Poren irgendwann vollständig gefüllt sein werden und der Washcoat unzugänglich werden wird.

Monolithe bieten günstige Voraussetzungen für die Durchführung der autothermen Dehydrierung von Kohlenwasserstoffen: insbesondere sind engere Reaktorquerschnitte und höhere Strömungsgeschwindigkeiten gegenüber regellos gepackten Festbetten realisierbar, so dass eine effektive, gestufte Zudosierung des Sauerstoffes in den Kohlenwasserstoff enthaltenden Hauptstrom möglich ist. Die Hauptströmungsrichtung durch den Reaktor ist nicht auf eine Abwärtsströmung begrenzt, wie im Fall von regellos gepackten Festbetten.

Nach längerer Standzeit können die in der vorliegenden Schrift empfohlenen Katalysatoren normalerweise auf einfache Art und Weise regeneriert werden, beispielsweise indem man zunächst in ersten Regenerationsstufen Luft, die (vorzugsweise) mit Stickstoff und/oder Wasserdampf verdünnt ist, bei einer Eintrittstemperatur von 300 bis 600 °C (in Extremfällen auch bis zu 750 °C), häufig von 500 bis 600 °C, durch das Katalysatorfestbett leitet. Die Katalysatorbelastung mit Regenerationsgas kann (bezogen auf die Gesamtmenge an regeneriertem Katalysator) beispielsweise 50 bis 10 000 $h^{-1}$ betragen, und der Sauerstoffgehalt des Regenerationsgases kann 0,5 bis 20 Vol-% betragen.

Danach ist es im Allgemeinen empfehlenswert, auch unter ansonsten identischen Bedingungen mit reinem molekularem Wasserstoff oder mit molekularem Wasserstoff, der mit Inertgas (vorzugsweise Wasserdampf und/oder Stickstoff) verdünnt ist, zu regenerieren (der Wasserstoffgehalt sollte ≥ 1 Vol.-% sein).

Die nebeneinander, übereinander und hintereinander zu einer Packung gestapelten Monolithe sind bevorzugt in einer Blähmatte oder in einem Mineralfaservlies eingehüllt und in eine Einhausung mit Verspanneinrichtung eingesetzt. Als Mineralfaservliese werden bevorzugt Vliese eingesetzt, wie sie für den Einsatz für Abgaskatalysatoren bekannt sind, beispielsweise Interam® Lagermatten der Firma 3M®.

Blähmatten sind aus der katalytischen Abgasreinigung bekannt und beispielsweise in DE-A 40 26 566 beschrieben: Sie bestehen im Wesentlichen aus Keramikfasern mit Glimmereinlagerung. Infolge der Glimmereinlagerung hat die Blähmatte bei steigenden Temperaturen das Bestreben sich auszudehnen, wodurch eine besonders sichere Halterung des darin eingehüllten Körpers auch bei höheren Temperaturen erreicht wird.

Die Mineralvliese oder Blähmatten werden so ausgewählt, dass sie sich unter Wärmeeinwirkung ausdehnen und dass sie die in der Regel keramischen Monolithe gegen das Gehäuse abdichten, insbesondere eine Reibung der Monolithe am Gehäuse sowie eine Bypassströmung des Reaktionsgasgemisches an der Innenwand des Gehäuses verhindern.

Die Blähmatten, in die die Monolithe eingehüllt sind, sorgen für eine stabile Position derselben, da sie unter Wärmeausdehnung eine Spannkraft erzeugen. Im Zuge von Fehlbedingungen kann die Spannkraft jedoch nachlassen. Daher kann vorteilhaft eine Verspanneinrichtung vorgesehen werden: Hierzu werden die Blähmatten an ihrem dem Austritt des Reaktionsgasgemisches entsprechenden Ende mit einem aus einem hochtemperaturfesten Gewebe, das beispielsweise metallisch sein kann, gebildeten U-Profil eingefasst. In Verlängerung der Blähmatten werden Metallprofile angeordnet, die mit einem Querschnitt entsprechend dem Querschnitt der Blähmatten an denselben ansetzen und sich in Strömungsrichtung des Reaktionsgasgemisches zunehmend verbreitern. Dadurch wirken die Metallprofile als Stütze gegen das Verschieben der Blähmatten in Strömungsrichtung des Reaktionsgasgemisches.

Die in Blähmatten eingehüllten Monolithe sind in einem Gehäuse angeordnet.

Das Gehäuse ist vorteilhaft aus einem Werkstoff gebildet, der bei der hohen Belastung durch die Reaktionstemperatur, häufig im Bereich von etwa 400 bis 700 °C, mechanisch und chemisch stabil ist und auch keine katalytische Aktivität für die autotherme Gasphasendehydrierung aufweist.

Bevorzugt ist das Gehäuse aus einem Werkstoff gebildet, der hitzebeständig ist, insbesondere aus einem Edelstahl mit der Werkstoffnummer 1.4541, 1.4910 oder 1.4841.

Das Gehäuse soll möglichst dünn sein, um eine möglichst geringe Wärmekapazität zu haben, und somit die Wärmeverluste zwischen dem Außenbereich B und dem Innenbereich A zu begrenzen.

Das Gehäuse kann bevorzugt thermisch isoliert sein.

Das Gehäuse kann bevorzugt lose im Reaktor gelagert sein.

Das Gehäuse ist bevorzugt als Quader ausgebildet.

Bevorzugt sind die Seitenwände des als Quader ausgebildeten Gehäuses einzeln abnehmbar ausgebildet, dergestalt, dass eine komplette Packung oder einzelne Monolithe einer Packung aus einer katalytisch aktiven Zone ausgetauscht werden können.

Erfindungsgemäß werden die einzelnen Monolithe nebeneinander, übereinander und hintereinander, in der erforderlichen Anzahl, um eine katalytisch aktive Zone auszufüllen, unter Ausbildung einer Packung, gestapelt.

Vor jeder Packung ist jeweils eine Mischzone mit festen Einbauten, die nicht katalytisch aktiv sind, vorgesehen. In der Mischzone erfolgt die Vermischung des kohlenwasserstoffhaltigen Gasstromes mit dem Sauerstoff enthaltenden Strom, wobei in der in Strömungsrichtung zuerst angeströmten Mischzone die Vermischung des Sauerstoff enthaltenden Gasstromes mit dem kohlenwasserstoffhaltigen Einsatzstrom erfolgt und in den darauffolgend angeströmten Mischzonen jeweils eine Zwischenspeisung eines Sauerstoff enthaltenden Gasstromes in das noch zu dehydrierende, Kohlenwasserstoff enthaltende Reaktionsgemisch erfolgt.

Der zu dehydrierende kohlenwasserstoffhaltige Gasstrom kann bevorzugt an zwei oder mehreren Stellen in den Wärmetauscher eingeleitet werden, insbesondere als ein Hauptstrom mit höherem Massenstrom und ein oder mehrere Nebenströme mit niedrigerem Massenstrom gegenüber dem Hauptstrom.

Für die Aufheizung des zu dehydrierenden kohlenwasserstoffhaltigen Gasstromes können zusätzlich zum Wärmetauscher eine oder mehrere Zusatzheizungen vorgesehen sein. Als Zusatzheizung kann bevorzugt die Zuführung von Wasserstoff durch die Zuführleitung für den zu dehydrierenden kohlenwasserstoffhaltigen Gasstrom möglichst nahe am Eintritt in die Mischzonen, die vor jeder katalytisch aktiven Zone angeordnet sind, vorgesehen sein.

Alternativ oder zusätzlich kann als Zusatzheizung eine elektrische Heizung vorgesehen sein, die bevorzugt lösbar, als Einstecksystem, innerhalb des Außenbereichs B des Reaktors in die Zuführleitung für den kohlenwasserstoffhaltigen Gasstrom, nach dem Austritt desselben aus dem Wärmetauscher eingebracht wird. Alternativ oder zusätzlich kann als Zusatzheizung ein Muffelbrenner vorgesehen sein.

Durch die Ausbildung des Reaktors als liegender Zylinder ist der Innenraum A, der die Monolith-Packungen umfasst, großflächig abgestützt und dadurch mechanisch entlastet. Darüber hinaus ist bei dieser Reaktoranordnung die Zugänglichkeit zu den einzelnen Monolithpackungen einfacher.

Der Mantel des Reaktors ist bevorzugt aus einem für Druckbehälter zugelassenen legierten Stahl gebildet, insbesondere aus Schwarzstahl, bevorzugt aus Kesselblech HII, oder aus einem legierten Stahl mit der Werkstoffnummer 1.4541 oder 1.4910. Der Mantel des Reaktors kann auch mit Schamotteausmauerung ausgekleidet sein.

Jede Mischzone umfasst bevorzugt jeweils einen Rohrverteiler, gebildet aus einer Vielzahl von parallel zueinander, in einer Ebene senkrecht zur Längsrichtung des Reaktors angeordneten Einsteckrohren, die mit einer oder mehreren der Verteilerkammern verbunden sind, und die eine Vielzahl von gleichmäßig zueinander beabstandeten Austrittsöffnungen für den Sauerstoff enthaltenden Gasstrom aus dem Einsteckrohr aufweisen, sowie eine Vielzahl von gleichmäßig zueinander beabstandeten Mischkörpern.

Die Mischkörper können vorteilhaft als Mischplatten ausgebildet sein.

Der Wärmetauscher ist bevorzugt ein Rohrbündelwärmetauscher.

Der Rohrbündelwärmetauscher ist vorteilhaft aus einem hoch warmfesten Edelstahl, insbesondere einem Edelstahl mit der Werkstoffnummer 1.4541 oder 1.4910, gebildet. Die Rohre des Rohrbündelwärmetauschers sind an beiden Enden derselben in Rohrböden vorteilhaft spaltfrei durch Hinterbodenschweißung eingebracht und die Rohrböden des Rohrbündelwärmetauschers auf der Heißgasseite derselben mit einem hitzebeständigen Edelstahl, insbesondere mit einem Edelstahl mit der Werkstoffnummer 1.4841, plattiert.

Bevorzugt ist an der Stirnseite des Gehäuses G, an der der kohlenwasserstoffhaltige Gasstrom in den Innenbereich A eingeleitet wird, ein Strömungsgleichrichter angeordnet.

Gegenstand der Erfindung ist auch ein Verfahren zur Durchführung von autothermen Dehydrierungen unter Verwendung des vorstehend beschriebenen Reaktors.

In einer bevorzugten, voll kontinuierlichen Fahrweise können zwei oder mehrere Reaktoren eingesetzt werden, wobei mindestens ein Reaktor für die autotherme Gasphasendehydrierung genutzt und gleichzeitig mindestens ein weiterer Reaktor regeneriert wird.

Bevorzugt wird die Regenerierung in einem Temperaturbereich zwischen 550 und 700° C durchgeführt.

Weiter bevorzugt wird die Regenerierung mit einem Sauerstoff enthaltenden Gasstrom mit einem Sauerstoffgehalt zwischen 0,1 und 1,5 Gew.-% Sauerstoff, bezogen auf das Gesamtgewicht des Sauerstoff enthaltenden Stromes, durchgeführt.

Um den Raffinat II-Prozess wirtschaftlich zu betreiben, ist eine periodische Regeneration des verkokten Katalysators vorteilhaft. Im Rahmen der Vorarbeiten wurde der periodische Betrieb in einer Miniplant vorgefahren und eine Regenerationsprozedur entwickelt, die eine zyklenstabile Produktivität ermöglicht. Die Dehydrierungs- und die Regenerationsphase dauern jeweils 12 Stunden. Damit ist eine quasi kontinuierliche Produktion mit zwei abwechselnd betriebenen Reaktoren möglich.

Zu Beginn des Regeneriervorgangs befindet sich der Katalysator auf der Betriebstemperatur und dem Betriebsdruck beim Abstellen der Dehydrierung. Im Gasraum ist noch das Dehydriergasgemisch eingeschlossen. In einem Spülschritt wird das Reaktionsgemisch durch einen Stickstoffstrom ausgetrieben.

Anschließend wird der Koks abgebrannt. Dazu wird Magerluft, verdünnt mit Stickstoff, über den Katalysator geleitet. Das Abbrennen der Koksablagerungen erfordert eine rigide Temperaturkontrolle, da der Abbrennvorgang innerhalb eines Temperaturfensters zwischen 550 und 700°C ablaufen muss: Bei tieferen Temperaturen bleibt eine Restbeladung auf dem Katalysator zurück. Zu hohe Temperaturen beschleunigen die irreversible thermische Katalysatordesaktivierung. Der Abbrennvorgang wird anhand der Ausbildung einer langsam in Strömungsrichtung wandernden Temperaturfront und der Bildung von $CO_2$ erkennbar. Am Ende des Abbrennens bricht der Sauerstoff durch und die Sauerstoffkonzentration steigt an. Damit findet ein gleitender Übergang zur Redispergierungsphase statt.

Das Redispergieren des Katalysators soll die durch Koaleszenz der Katalysatorkristallite hervorgerufene Desaktivierung reparieren. Dazu wird Luft mit einer Temperatur von ca. 550°C über den Katalysator geleitet. Es wird vermutet, dass dadurch die aktive Komponente (eine Pt/Sn-Legierung) oxidiert wird und als flüssiger Oxidfilm den Träger benetzt. Die Dauer der Redispergierung richtet sich nach der Dauer des kompletten Regenerationszyklusses.

Nach der Redispergierung muss der Katalysator durch Reduktion wieder aktiviert werden. Gleichzeitig wird der Reaktor für die nachfolgende Produktionsphase konditioniert. Dieser Vorgang umfasst folgende Schritte:

a. Der Sauerstoff wird aus dem Reaktor ausgetrieben. Dazu wird der Reaktor mit Stickstoff oder $H_2O$-Dampf bei ca. 500°C gespült.
b. Die Katalysatorreduktion erfolgt durch ein Gemisch aus Wasserdampf und Wasserstoff im Volumenverhältnis 15:85 bei ca.500°C.
c. Die Dehydrierung wird gestartet.

Die autotherme Gasphasendehydrierung ist bevorzugt eine Dehydrierung von Propan, von Buten, von Isobuten, von Buten zu Butadien oder von Ethylbenzol zu Styrol.

Der erfindungsgemäße Reaktor und das erfindungsgemäße Verfahren weisen insbesondere die Vorteile auf, dass eine optimierte Reaktoranordnung bezüglich der mechanischen Belastung, der Handhabung sowie der Verbindung mit Peripherieapparaten, eine sichere Beherrschung der brennbaren Reaktionsmedien unter Vermeidung von Temperaturspitzen und entsprechender Werkstoffbelastung sowie eine einfache Zugänglichkeit und Handhabung der einzelnen Monolithe gewährleistet wird. Darüber hinaus ermöglicht die erfindungsgemäße Reaktoranordnung eine optimale Haupt- und Zwischeneinspeisung von Sauerstoff.

Besonders bevorzugt ist die autotherme Gasphasendehydrierung die Dehydrierung von Butan, wobei zwei wie vorstehend beschriebene Reaktoren eingesetzt werden, wobei ein Reaktor für die autotherme Gasphasendehydrierung genutzt und gleichzeitig ein zweiter Reaktor regeneriert wird.

Durch die Butandehydrierung wird Raffinat II, enthaltend in der Regel 70 Gew.-% Butene und 30 Gew.-% n-Butane, hergestellt.

Die Hauptreaktionen werden durch die folgenden stöchiometrischen Gleichungen beschrieben:

$$C_4H_{10} \rightarrow C_4H_8 + H_2 \qquad \Delta h_{R1}^0 = 122{,}8\, \tfrac{kJ}{mol} \qquad (1)$$

$$H_2 + \tfrac{1}{2}O_2 \rightarrow H_2O \qquad \Delta h_{R2}^0 = -246{,}2\, \tfrac{kJ}{mol} \qquad (2)$$

Als Katalysator wird ein Pt/Sn-System eingesetzt.

Unter technisch relevanten Bedingungen erreicht die Hauptreaktion (1) Teilumsatz. Das nicht umgesetzte Butan und ein Teil des Dehydrierwasserstoffs müssen aus dem Produktstrom abgetrennt und in den Reaktor zurück geführt werden. Deshalb ist die Reaktionsstufe mit einer Sequenz von Aufarbeitungsschritten gekoppelt.

Der Reaktor ist als Hordenreaktor mit drei katalytisch aktiven Schichten konzipiert. Der kohlenwasserstoffhaltige Gasstrom (Feedstrom) enthält Frischbutan, Wasserdampf sowie einen butanreichen und einen wasserstoffreichen Rücklauf aus der Aufarbeitung. Vor jeder katalytisch aktiven Zone befindet sich eine Seiteneinspeisung, wodurch Sauerstoff, verdünnt mit Wasserdampf, in den Hauptstrom zugemischt wird. In einer engen Zone am Eintritt der katalytisch aktiven Schicht wird das Reaktionsgemisch durch selektive Teilverbrennung des Dehydrierwasserstoffs aufgeheizt. Im weiteren Verlauf wird die Wärme von der Dehydrierungsreaktion verbraucht und das Reaktionsgemisch kühlt sich ab. Dadurch ergibt sich entlang des Reaktors ein sägezahnförmiges Temperaturprofil zwischen 500 und 650 °C.
Der optimale Betriebsdruck liegt bei 1,5 bis 2 bar absolut.

Unter diesen Bedingungen erreicht die Zielreaktion (1) einen Umsatz von ca. 40 % und eine Selektivität > 95 %. Die wichtigsten Nebenreaktionen sind:

Totaloxidation von Butan:

$$C_4H_{10} + \frac{13}{2}O_2 \rightarrow 4CO_2 + 5H_2O \quad \Delta H_R(550°C) = -2657\frac{kJ}{mol} \qquad (3)$$

Diverse Spaltreaktionen:

$$C_4H_{10} \rightarrow C_3H_6 + CH_4 \quad \Delta H_R(550°C) = 67,2\frac{kJ}{mol} \qquad (4)$$

$$C_4H_{10} \rightarrow C_2H_6 + C_2H_4 \quad \Delta H_R(550°C) = 90,2\frac{kJ}{mol} \qquad (5)$$

Produktdehydrierung:

$$C_4H_8 \rightarrow C_4H_6 + H_2 \quad \Delta H_R(5550°C) = 179,8\frac{kJ}{mol} \qquad (6)$$

Die Dehydrierung der adsorbierten Kohlenwasserstoffe kann darüber hinaus bis zur Verkokung voranschreiten, formal:

$$C_4H_8 \rightarrow 4C + 4H_2 \quad \Delta H_R(550°C) = 32,7\frac{kJ}{mol} \qquad (7)$$

Weitere Nebenreaktionen, ohne direkte Auswirkung auf die Produktselektivität, sind die Wassergas-Shift-Reaktion:

$$CO + H_2O \rightarrow CO_2 + H_2 \quad \Delta H_R(550°C) = -36,6\frac{kJ}{mol} \qquad (8)$$

und die Bouduard-Reaktion:

$$2CO \rightarrow CO_2 + C \quad \Delta H_R(550°C) = -171,3\frac{kJ}{mol} \qquad (9)$$

Unter den gegebenen Betriebsbedingungen wird der Katalysator innerhalb weniger Stunden mit Koksablagerunen belegt und verliert seine Anfangsaktivität. Die Verkokung macht sich durch den Anstieg des Temperaturniveaus bemerkbar.

Um das Verfahren wirtschaftlich zu betreiben, ist daher eine periodische Regeneration des verkokten Katalysators erforderlich.

Der Reaktor wird im Produktionsmodus der autothermen Gasphasendehydrierung gefahren, bis ein normaler Reaktorbetrieb auf Grund der Verkokung des Katalysators nicht mehr möglich ist, worauf der Reaktor in den Regeneriermodus geschaltet wird. Gleichzeitig wird ein zweiter, gleichartiger Reaktor vom Regeneriermodus in den Produktionsmodus der autothermen Gasphasendehydrierung geschaltet.

Insbesondere werden der Produktionsmodus sowie der Regeneriermodus über jeweils 12 Stunden gefahren. Damit ist eine quasi kontinuierliche Fahrweise mit zwei abwechselnd betriebenen Reaktoren möglich.

Die Regenerierung umfasst die folgenden Verfahrensschritte:

1. Inertisieren (Spülen) des Reaktors

Zu Beginn des Regeneriermodus befindet sich der Katalysator auf der Betriebstemperatur und dem Betriebsdruck beim Abstellen der autothermen Gasphasendehydrierung. Im Gasraum ist noch des Dehydriergasgemisch eingeschlossen. Im ersten Regenerationsschritt (Inertisieren bzw. Spülen) wird das Reaktionsgasgemisch durch insbesondere einen Stickstoffstrom ausgetrieben.

2. Abbrennen

In einem nächsten Regenerierschritt, dem Abbrennen der Koksablagerungen an der Oberfläche des heterogenen Katalysators, wird Magerluft, verdünnt mit Stickstoff, über den Katalysator geleitet. Das Abbrennen der Koksablagerungen erfordert eine rigide Temperaturkontrolle, da der Abbrennvorgang innerhalb eines Temperaturfensters zwischen 550 und 700°C ablaufen muss: Bei tieferen Temperaturen bleibt eine Restbeladung auf dem Katalysator zurück. Zu hohe Temperaturen beschleunigen die irreversible thermische Katalysatordesaktivierung. Der Abbrennvorgang wird anhand der Ausbildung einer langsam in Strömungsrichtung wandernden Temperaturfront und der Bildung von $CO_2$ erkennbar. Am Ende des Abbrennens bricht der Sauerstoff durch und die Sauerstoffkonzentration steigt an. Damit findet ein gleitender Übergang zur Redispergierungsphase statt.

3. Redispergieren

Das Redispergieren (Verfahrensschritt 3) des heterogenen Katalysators soll die durch Koaleszenz der Katalysatorkristallite hervorgerufene Desaktivierung rückgängig machen. Dazu wird Luft mit einer Temperatur von ca. 550 °C über den Katalysator geleitet. Es wird vermutet, dass dadurch die aktive Komponente (eine Pt/Sn-Legierung) oxidiert wird und als flüssiger Oxidfilm den Träger benetzt. Die Verteilung des Katalysators auf der Trägeroberfläche wird über den Dispersionsgrad angegeben. Der Dispersionsgrad ist definiert als der Anteil der Atome der aktiven Substanz mit direktem Kontakt zur Oberfläche. Demnach wäre der Dispersionsgrad einer Monolage 100 %. Der Dispersionsgrad ist nicht direkt messbar und kann nur über die Performance des Katalysators im Dehydrierzyklus beurteilt werden.

4. Spülen

An das Redispergieren schließt sich im nächsten Verfahrensschritt ein Spülen des heterogenen Katalysators an und schließlich ein weiterer Verfahrensschritt, das

5. Reduzieren des heterogenen Katalysators, um diesen wieder zu aktivieren.

Gleichzeitig wird der Reaktor für die nachfolgende Produktionsphase konditioniert. Dieser Vorgang umfasst folgende Schritte:

    a. Der Sauerstoff wird aus dem Reaktor ausgetrieben. Dazu wird der Reaktor mit Stickstoff oder $H_2O$-Dampf bei ca. 500°C gespült.

    b. Die Katalysatorreduktion erfolgt durch ein Gemisch aus Wasserdampf und Wasserstoff im Volumenverhältnis 15:85 bei ca.500°C.

    c. Die Dehydrierung wird gestartet.

Im Folgenden werden detailliert die relevanten Betriebszustände für einen Reaktor zur autothermen Gasphasendehydrierung von Butan beschrieben:

    1. Erstinbetriebnahme des Reaktors
    2. Anfahren des Reaktors nach einem Stillstand

3. Bestimmungsgemäßer, zyklischer Betrieb mit den folgenden Betriebsmodi:

  3.1 Produktionsmodus
  3.2 Regeneriermodus

    a. Spülen
    b. Abbrennen
    c. Oxidieren / Redispergieren
    d. Spülen
    e. Reduzieren

  4. Abfahren des Reaktors
  5. Notabschaltung bei nicht bestimmungsgemäßem Betrieb.

1. Erstinbetriebnahme bzw. 2. Anfahren des Reaktors nach einem Stillstand

Die Anfahrprozedur soll den Anfangszustand für den periodischen Betrieb etablieren. Sie startet, wenn der Reaktor, die Zuleitungen und die Abführleitung vorgeheizt und mit Wasserdampf geflutet sind. Dafür ist hauptsächlich die Einstellung eines geeigneten Temperaturniveaus in der Reaktionsstrecke, d.h. in den katalytisch aktiven Zonen, erforderlich.

Hierzu wird im Wärmeübertrager der Zulauf von 200°C auf ca. 500 °C aufgeheizt. Die Temperatur in der Reaktionsstrecke liegt bei 550 °C.

Insbesondere sind zwei Möglichkeiten zur Vorheizung der Reaktionsstrecke vorgesehen:

- das reaktive Aufheizen durch katalytische Verbrennung von Wasserstoff:

  Wasserstoff wird als Brenngas verwendet, da die Wasserstoffverbrennung ab der Vorheiztemperatur spontan am Pt/Sn-Katalysator zündet. Der Reaktor wird im Kreislaufmodus betrieben. Über den Hauptstrom wird Luft in den Kreislauf eingeleitet. Über den Seitenstrom wird Wasserstoff eingeleitet und mit dem Hauptstrom vermischt in einem stöchiometrischen Verhältnis von Sauerstoff zu Wasserstoff, das einen vollständigen Verbrauch des Wasserstoffs gewährleistet. Die Aufheizprozedur dauert ca. zwölf Stunden. Am Ende dieser Phase hat die Reaktionsstrecke ein Temperaturniveau von 550 °C erreicht.

- Stützheizung im Hauptstrom:

  In der Leitung zum Eintritt in die Reaktionsstrecke ist ein Anschluss für eine Stützheizung vorgesehen. Bei Bedarf kann in diesen Leitungsabschnitt eine elektrische Heizung, ein Muffelbrenner oder ein Rekuperativbrenner installiert werden. Über die Hauptleitung wird ein kleiner Stickstoffstrom in den Kreislauf eingeleitet.

Die reaktive Aufheizung hat den Vorteil, dass die Wärme direkt am Katalysator freigesetzt wird. Dadurch können Wärmeverluste minimiert werden. Außerdem erfordert die Prozedur keinen zusätzlichen apparativen Aufwand. Die Verwendung von Wasserstoff ist vorteilhaft, weil seine katalytische Verbrennung ab 200°C spontan und ohne Stützheizung startet. Nachteilig ist, dass eine Fehldosierung des Brennstoffs zur Überhitzung des Katalysators, schlimmstenfalls auch zur Bildung eines zündfähigen Gemisches führen kann. Hinzu kommt, dass die reaktive Aufheizung einen hinreichend aktiven Katalysator voraussetzt. Diese Voraussetzung ist jedoch nicht gesichert, beispielsweise wenn der Katalysator stark verkokt ist.

Der Einsatz einer externen Stützheizung (Elektroheizung oder Brenner) erhöht die Zuverlässigkeit der Prozedur. Speziell die elektrische Vorheizung bietet den Vorteil, dass der Katalysator in einer schonenden, inerten Atmosphäre auf Reaktionstemperatur vorgeheizt werden kann. Der regelungstechnische und sicherheitstechnische Aufwand wird dadurch erheblich reduziert. Nachteil der externen Stützheizung ist der zusätzlich erforderliche apparative Aufwand sowie die Betriebskosten (gilt für die Elektroheizung). Ein prozesstechnischer Nachteil ergibt sich aus der Distanz zwischen der Heizquelle und dem Katalysator. Die Wärmeverluste im dazwischen liegenden Leitungsabschnitt müssen kompensiert werden, um die angestrebte Katalysatortemperatur zu erreichen.

An die Aufheizungsphase schließt sich eine Konditionierungsphase an. Diese startet, wenn die Reaktionsstrecke auf 550 °C aufgeheizt, im Wärmeübertrager ein stationäres, annähernd lineares Temperaturprofil zwischen der Zulauftemperatur und der Reaktionstemperatur etabliert und das Reaktorvolumen mit Inertgas (Stickstoff oder Wasserdampf) geflutet ist.

Die Konditionierungsprozedur soll den Katalysator vor Beginn des bestimmungsgemäßen Betriebs in einen definierten, aktiven Zustand überführen. Die Konditionierung umfasst eine Reduktion-Oxidation-Reduktion-Sequenz (auch als ROR-Prozedur bezeichnet). Der erste Reduktionsschritt dient hauptsächlich dazu, flüchtige Pt-Salze zu reduzieren. Diese Salze, insbesondere Platin(IV)-Chlorid können Rückstände aus der Katalysatorpräparation sein. Im Oxidationsschritt werden mögliche organische Ablagerungen von der Katalysatoroberfläche weggebrannt und die Katalysatorkristallite redispergiert.

Im anschließenden Reduktionsschritt wird der Katalysator aktiviert.

3.1 Produktionsmodus

Im Anfangszustand liegt die Temperatur in der Reaktionsstrecke bei ca. 550 °C, im Wärmeübertrager ist ein annähernd lineares Temperaturprofil zwischen der Zulauftemperatur und der Reaktionstemperatur eingestellt. Der Katalysator ist in seinem reduzierten, aktiven Zustand und frei von Koksablagerungen. Das Reaktionsvolumen ist mit Wasserstoff/Wasserdampf gefüllt. Der Druck beträgt 1,5 bar(a), gemessen am Reaktoraustritt.
Der Reaktor wird im einfachen Durchgang betrieben. Im Hauptstrom 4001 wird das Edukt der Dehydrierung zugeführt. Neben den aufgeführten Komponenten können optional Spurenkomponenten zugegeben werden. Die folgende Tabelle gibt den Bereich an, in dem die Konzentration der Spurenkomponenten variiert werden soll.

Konzentrationsbereich der Spurenkomponenten im Eduktstrom während der Dehydrierung

| Komponente | Volumenanteil |
|---|---|
| $C_4H_6$ | 0 - 0,5 % |
| $CH_4$ | 0 - 2,0 % |
| CO | 0 - 5,0 % |
| CO | 0 - 0,5 % |
| NMP | 0 - 500 ppm |

Entsprechend der Zumischung wird sich der Anteil der Hauptkomponenten reduzieren. Durch die Seiteneinspeisungen werden die zur Wärmeversorgung der autothermen Dehydrierung benötigten, sauerstoffhaltigen Ströme eingeleitet. Der Sauerstoffanteil beträgt 15 Vol.-% in einem Trägerstrom aus Wasserdampf.
Der Sollwert für den Druck am Austritt der Reaktionsstrecke wird auf 1,5 bar(a) gesetzt. Bei Nennlast ergibt sich folgender Druckverlaüf über der Reaktionsstrecke (jeweils am Eintritt der katalytisch aktiven Zone):

1. katalytisch aktive Zone:  2,0 bar
2. katalytisch aktive Zone:  1,87 bar
3. katalytisch aktive Zone:  1,70 bar

Im Wärmeübertrager ist der rohrseitige (produktseitige) Druckabfall < 15mbar, ist also vernachlässigbar gegenüber dem Druckabfall in der Reaktionsstrecke.
Das Temperaturprofil in der katalytisch aktiven Zone zeigt den typischen sägezahnförmigen Verlauf, bedingt durch die Zwischeneinspeisung von Sauerstoff. Im Wärmeübertrager stellt sich aufgrund des höheren Wärmekapazitätsstroms auf der Produktseite gegenüber der Feedseite ein leicht konvexer Temperaturverlauf ein. Das Temperaturniveau steigt während der Dehydrierungsphase kontinuierlich an. Ursache dafür ist die Akkumulation von Kohlenstoffablagerungen am Katalysator. Die Kohlenstoffablagerungen führen zur Deaktivierung des Katalysators, wodurch der Wärmeverbrauch der Dehydrierungsreaktion tendenziell absinkt. Folglich steigt die Austrittstemperatur aus der katalytisch aktiven Zone und damit auch die treibende Temperaturdifferenz im Wärmeübertrager an. Das bewirkt eine Anhebung der Vorheiztemperatur. Durch den Anstieg des Temperaturniveaus wird der Aktivitätsverlust weitgehend kompensiert. So hat die Deaktivierung nur einen geringen Einfluss auf den Endumsatz. Dieses selbstadaptierende Verhalten des wärmeintegrierten Systems vereinfacht die Prozessführung substanziell, da keine Nachführung der Sauerstoffmenge zur Erhaltung der Produktionsrate erforderlich ist.

Bei Lastvariationen müssen folgende Kriterien beachtet werden:

- Strömungstechnische Limitierung nach oben:

  o Druckabfall in der Reaktionsstrecke: $\Delta p_{1001 \rightarrow 1010}$ < 700 mbar
  o Geschwindigkeit im engsten Querschnitt: $V_{max}$ < 60 m/s

- Strömungstechnische Limitierung nach unten:

    o Verweilzeit in der Mischzone: $\tau_{mix}$ < 100 ms
    o Re-Zahl in der Mischzone: Re > 6000
    o

- Temperaturlimitierung in den Mischzonen: $T_{mix}$ < 550 °C

- Limitierung durch Wärmeverluste: $\Delta T_{loss}$ < 20 K

$\Delta T_{loss}$ bezeichnet dabei die Absenkung der Temperatur am Austritt der Reaktionsstrecke gegenüber einer adiabaten Messstrecke.

Die strömungstechnischen Limitierungen ergeben sich aus der Reaktorkonstruktion. Die Temperaturlimitierung in den Mischzonen ist festgelegt, um Selektivitätsverluste durch das Zünden der homogenen Verbrennung zu vermeiden. Die Limitierung der Wärmeverluste soll Verfälschungen des Betriebszustandes begrenzen.

Bei gegebener Zusammensetzung der Feedströme ergeben obige Limits folgenden erlaubten Lastbereich für den Reaktor:

| Strom | | MIN | MAX |
|---|---|---|---|
| Hauptstrom (4001) | | 540 kg/h (350 Nm$^3$/h) | 925 kg/h (600 Nm$^3$/h) |
| Seiten-ströme | 5004 | 77 kg/h (86 Nm$^3$/h) | 132 kg/h (148 Nm$^3$/h) |
| | 5002 | 44 kg/h (49 Nm$^3$/h) | 76 kg/h (85 Nm$^3$/h) |
| | 5003 | 32 kg/h (36 Nm$^3$/h) | 55 kg/h (62 Nm$^3$/h) |

3.2 Regeneriermodus

Die periodische Regeneration des Katalysators ist eine komplexe Prozedur, die mehrere Phasen umfasst. Die zeitliche Abfolge der Regenerationsschritte ist in der folgenden Tabelle dargestellt.

| Phase | Dauer (ca.) | Betriebsmodus |
|---|---|---|
| a. Spülen (Inertisieren) | 5 min | Einfacher Durchgang |
| b. Abbrennen | 4 h 30 min | Kreislaufführung |
| c. Redispergieren | 5 h 55 min | Kreislaufführung |
| d. Spülen | 30 min | Kreislaufführung |
| e. Reduzieren | 1 h | Kreislaufführung |

a. Spülen (Inertisieren)

Beim Inertisieren wird das Reaktorvolumen mindestens fünffach durch Stickstoff ausgetauscht. Dadurch sollen die Reste des Reaktionsgasgemisches der Dehydrierung ausgetrieben werden.

b. Abbrennen

Das Abbrennen des Kokses an der Katalysatoroberfläche ist der zentrale Regenerationsschritt, um die Katalysatoraktivität wieder herzustellen. Dieser Schritt stellt besondere Anforderungen an die Prozessregelung. Die regelungstechnische Aufgabe lässt sich wie folgt formuliert: Die Kohlenstoffbeladung soll in minimaler Zeit und auf minimalem Temperaturniveau eliminiert werden. Die grundsätzliche Schwierigkeit dieser Aufgabe ist, dass weder die Kohlenstoffbeladung des Katalysators noch die Abbrandgeschwindigkeit direkt messbar sind. Die Modellvorstellung darüber basiert primär auf Anpassungen an Miniplantexperimente. Weitere Hinweise geben TGA-Analysen von Ausbauproben verkokter Katalysatoren. Diese deuten auf zwei morphologisch oder stofflich unterschiedliche Arten von Koks hin. Im TGA-Diagramm startet die Verbrennung der ersten Fraktion bei ca. 550°C und weist die typische, exponentielle Beschleunigung über der Temperatur auf. Das Signal fällt bei 580°C abrupt ab und wird fortgesetzt mit einem wesentlich flacheren Verlauf bis ca. 630°C. Diese Beobachtung ist konsistent mit den Ergebnissen spektroskopischer Analysen für die Propandehydrierung an Modellkatalysatoren.

Die verfügbaren Informationen reichen nicht für einen wissensbasierten Reglerentwurf aus. Vielmehr wird eine heuristische Vorgehensweise verfolgt, wobei das Temperatur-profil und der Sauerstoffverbrauch als Ersatz-Regelgrößen herangezogen werden. Die Zuordnung von Regel- und Stellgrößen ist in der folgenden Tabelle zusammengefasst.

Regel- und Stellgrößen für das Abbrennen der Koksablagerungen

| Regelgrößen | Stellgrößen |
|---|---|
| Maximaltemperatur | Sauerstoffkonzentration |
| Dauer des Abbrennvorgangs | Sauerstoffmenge |
| Geschwindigkeit des Abbrennens | Katalysatortemperatur |
| Gleichverteilung der Strömung | Volumenstrom |

Simulationsstudien zeigen, dass die Regelgrößen in komplexer Weise von allen Stellgrößen beeinflusst werden. Außerdem ist die Maximaltemperatur, aufgrund der örtlichen Verteilung, nicht direkt steuerbar. Deshalb muss das Regelungskonzept weiter vergröbert werden: Der Abbrennvorgang erfolgt nicht mehr zustandskontrolliert sondern zeitkontrolliert.

Die einzige regelbare Größe ist die Eintrittstemperatur des Gasstroms in die Reaktionszone mit einem Sollwert:

$$T_{in}^{set} = 570°C\,.$$ Als Stellgrößen dafür dienen die Dosierung eines Stützgases, falls der Sollwert unterschritten wird und ein Bypassstrom um den Wärmeübertrager, falls der Sollwert überschritten wird. Die Sauerstoffmenge und die Sauerstoffkonzentration werden empirisch auf feste Werte eingestellt. Die Gasrückführung wird aktiviert.

Mit den gewählten Einstellungen ist es möglich, innerhalb von fünf Stunden die Kohlenstoffablagerungen komplett zu entfernen. Die erste und zweite Zone werden simultan regeneriert, während die Beladung in der dritten Zone zunächst weitgehend erhalten bleibt. Die Ablagerungen werden gleichmäßig über die Länge der jeweiligen Zone abgebaut. Nur gegen Ende der Regenerationsphase werden die Ablagerungen in der dritten Zone wellenförmig abgebaut.

Die positive Rückkopplung durch den integrierten Wärmerücktausch bewirkt ein anregbares Verhalten der Reaktionsstrecke. Die Maximaltemperatur kann nicht auf einen beliebigen Sollwert geregelt werden. Andererseits ist die Einhaltung eines Sollwertes für die Maximaltemperatur nicht erforderlich. Die Regeneration kann sauerstofflimitiert mit einer festen Sauerstoffzufuhr durchgeführt werden. Das Temperaturprofil verändert sich dabei wellenförmig, so dass Temperaturspitzen lokal nur kurzzeitig wirksam sind. Eine Überhitzung des Katalysators wird sinnvollerweise durch eine Schaltung bei Überschreitung einer oberen Temperaturschranke verhindert. Diese unterbricht die Sauerstoffzufuhr. Die Temperaturspitze muss in diesem Fall durch den inerten Trägergasstrom aus der Reaktionszone hinausgeschoben werden.

Die Kreislaufführung des Regenerationsgases hat mehrfache Vorteile für die Kontrolle des Abbrennvorgangs. Ein offensichtlicher Vorteil ist die Minimierung des Inertgasverbrauchs, um die geforderte Verdünnung des Sauerstoffs zu erreichen. Ein weiterer Vorteil ist die automatische Adaption der Sauerstoffkonzentration im Kreislauf. In der betrachteten Konfiguration variiert die Sauerstoffkonzentration zwischen 0,52 Gew.-% bei vollständigem Sauerstoffverbrauch und 6,97 Gew.-% bei vollständigem Erhalt des Sauerstoffs. Der Anstieg der Sauerstoffkonzentration begünstigt zu Beginn der Regeneration das Zünden der Koksablagerungen. Am Ende der Phase steigt die Sauerstoffkonzentration rampenförmig an und bewirkt eine kontinuierlichen Übergang zur anschließenden Redispergierung mit Vollluft.

c. Oxidieren (Redispergieren) des heterogenen Katalysators

Die Redispergierung des Katalysators wird mit Luft durchgeführt. Dazu wird ein Stützgas dosiert, um die Temperatur in der Reaktionszone zu regeln. Dabei können evtl. vorhandene Restablagerungen von der Katalysatoroberfläche entfernt werden. Die Dauer dieser Phase wird angepasst, um eine Synchronisierung zwischen dem Produktions- und dem Regeneriermodus zu erreichen. Diese Phase eignet sich auch für einen Stand-by Betrieb des Reaktors.

d. Spülen

Während der Spülphase wird der Reaktor inertisiert, um eine Vermischung sauerstoffhaltiger Gase aus der Redispergierungsphase und wasserstoffhaltiger Gase aus der nachfolgenden Reduzierphase auszuschließen.

e. Reduzieren

Die Hauptfunktion der Reduzierphase ist die Überführung der anoxidierten Katalysatorkomponenten in den metallischen, katalytisch aktiven Zustand. Gleichzeitig werden die Kristallite nach der Redispergierung in ihrer Form fixiert. Daneben muss das Temperaturprofil für den Start der Produktion eingestellt werden. Dabei wird in den katalytisch aktiven Zonen ein Temperaturniveau von ca. 500 °C angestrebt. Durch die integrierte Wärmerückführung ist die Abkühlung so langsam, dass im Endzustand das Temperaturniveau in den katalytisch aktiven Zonen noch deutlich oberhalb des Sollwertes liegt. Dieser Zustand kann durch eine Temperaturregelung korrigiert werden: Bei Überschreitung des Sollwertes kann der Bypass am Wärmeübertrager geöffnet werden. Bei Unterschreitung des Sollwertes kann über die Seiteneinspeisung Sauerstoff unterstöchiometrisch zudosiert werden, womit ein Teil des Wasserstoffs verbrannt wird.

Bei abgeschaltetem Kreisgaskühler steigt die Temperatur am kalten Ende des Wärmeübertragers auf ca. 350 °C an. Dieser Wert entspricht der Temperatur in der Rezirkulationsschleife. Dadurch droht die Überhitzung des Kreisgasgebläses. Dagegen wird bei eingeschaltetem Kreisgaskühler die Temperatur gezielt auf ca. 200 °C kontrolliert. So kann die thermische Belastung der Apparate und Armaturen im Kreislauf reduziert, bzw. die Spezifikation für diese Komponenten entschärft werden.

Die Erfindung ermöglicht es, autotherme Gasphasendehydrierungen mit niedrigeren Investitions- und Betriebskosten durchzuführen, sowie Monolithkatalysatoren mit höherer Last und verbesserter Selektivität für autotherme Gasphasendehydrierungen zu nutzen.

Die Erfindung wird im Folgenden anhand eines Ausführungsbeispiels sowie einer Zeichnung näher erläutert.

Es zeigen im Einzelnen:

Figur 1     einen Längsschnitt durch eine bevorzugte Ausführungsform eines erfindungsgemäßen Reaktors in horizontaler Ebene,

Figur 2     einen Längsschnitt durch denselben Reaktor in vertikaler Ebene,

Figur 3     einen Querschnitt durch den in Figur 2 dargestellten Reaktor in der Ebene B-B,

Figur 4     einen Querschnitt durch den in Figur 2 dargestellten Reaktor in der Ebene A-A und

Figur 5     eine vergrößerte Darstellung des in Figur 1 durch Einkreisung markierten Bereichs.

In den Figuren bezeichnen gleiche Bezugszeichen jeweils gleiche oder entsprechende Merkmale.

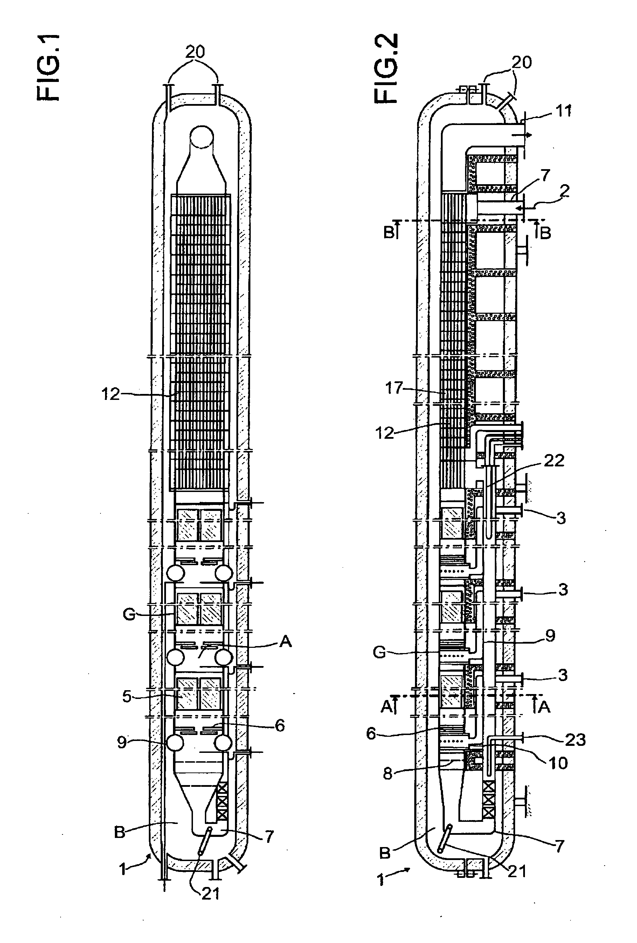

Der Längsschnitt in horizontaler Ebene in Figur 1 zeigt schematisch eine bevorzugte Ausführungsform eines erfindungsgemäßen Reaktors 1, der mit einem zu dehydrierenden kohlenwasserstoffhaltigen Gasstrom 2 über eine Zuführleitung 11 und über die Zuführleitungen 9 mit einem Sauerstoff enthaltenden Gasstrom 3 gespeist wird. Figur 1 zeigt, dass das Gehäuse G den Innenraum des Reaktors 1 in einen Innenbereich A und einen Außenbereich B aufteilt. Dem Innenbereich A schließt sich an einem Ende desselben ein Wärmetauscher 12 an. Auf der rechten Seite der Darstellung sind Zuführleitungen 20 für den Purge-Gasstrom und auf der linken Seite eine Verbindungsleitung 21 für den Purge-Gasstrom aus dem Außenbereich B des Reaktors in die Zuführleitung 7 für den zu dehydrierenden kohlenwasserstoffhaltigen Gasstrom 2 dargestellt. Über die Zuführleitungen 20 wird ein Purge-Gasstrom in den Außenbereich B eingeleitet, und über eine Verbindungsleitung 21 am anderen Ende des Reaktors über die Zuführleitung 7 für den zu dehydrierenden kohlenwasserstoffhaltigen Gasstrom 2 in den Innenbereich A weitergeleitet.

Die Figur zeigt Zusatzheizungen, die vorteilhaft eingesetzt werden können: eine elektrische Heizung 22 sowie eine Zuführleitung 23 für Wasserstoff als Brenngas in die Zuführleitung 7 für den zu dehydrierenden kohlenwasserstoffhaltigen Gasstrom 2.

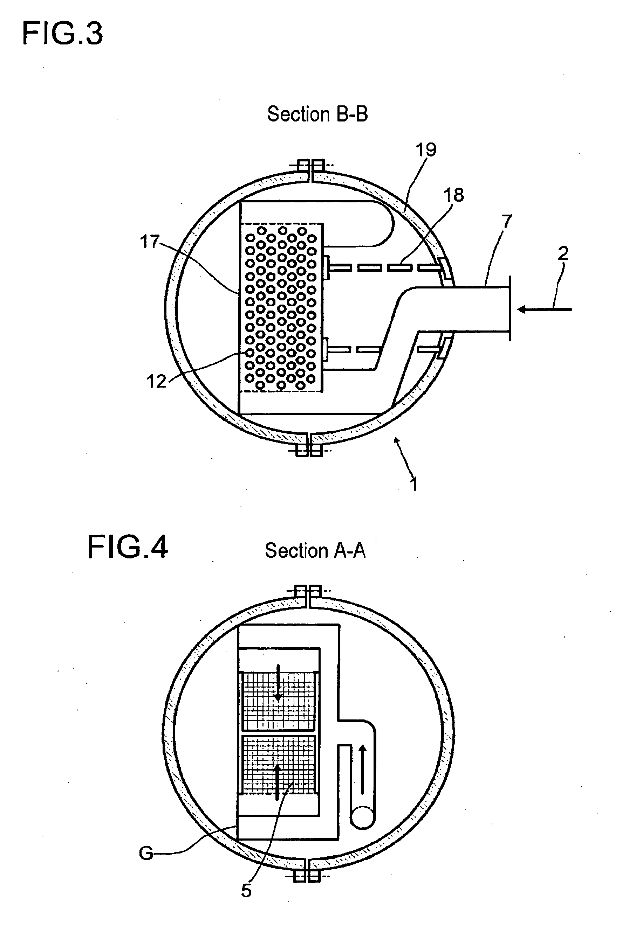

Figur 3 zeigt eine Querschnittdarstellung der Ebene B-B im Bereich des Wärmeübertragers. Die Figur verdeutlicht die Zuführung des zu dehydrierenden kohlenwasserstoffhaltigen Gasstromes 2 über die Zuführleitung 7 in den Zwischenraum zwischen den Rohren 17 der in der Figur dargestellten bevorzugten Ausführungsform eines Rohrbündelwärmetauschers 12.

In der Querschnittdarstellung ist ebenfalls die Tragekonstruktion 18 für den Rohrbündelwärmetauscher 12 zu erkennen, die bevorzugt aus Lochblechen gebildet ist, die Isolierschicht 19 der Innenwand des Reaktormantels sowie die bevorzugt als Zusatzheizung eingesetzte elektrische Heizung 22.

Figur 4 zeigt eine weitere Querschnittdarstellung in der Ebene A-A im Bereich einer Reaktionszone. Die Figur zeigt insbesondere die Zuführleitung 23 für Brenngas.

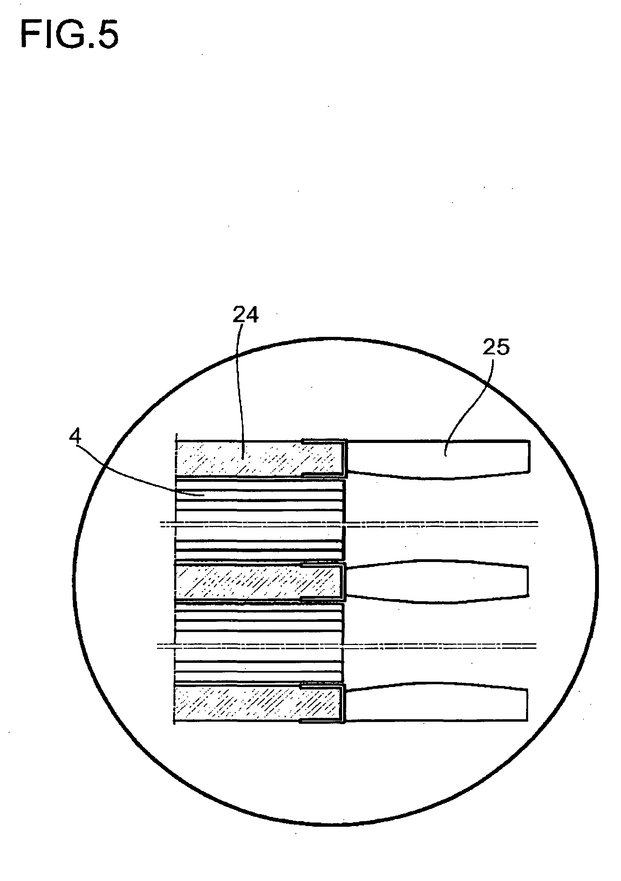

Der in Figur 5 dargestellte Ausschnitt verdeutlicht die in Verlängerung der Blähmatten 24, die in U-Profilen aus hochtemperaturfesten Geweben eingefasst sind, zugeordneten Stahlprofile 25, die mit einem Querschnitt entsprechend jenem der Blähmatten 24 an denselben ansetzen und sich zunehmend verbreitern. Die Bezugsziffer 4 in Figur 5 bezeichnet die Monolithe.

**Patentansprüche**

1. Reaktor (1) in Form eines liegenden Zylinders oder Prismas zur Durchführung einer autothermen Gasphasendehydrierung eines kohlenwasserstoffhaltigen Gasstromes (2) mit einem Sauerstoff enthaltenden Gasstrom (3) unter Erhalt eines Reaktionsgasgemisches, an einem heterogenen Katalysator, der als Monolith (4) ausgebildet ist, wobei

   - der Innenraum des Reaktors (1) durch ein lösbar, in Längsrichtung des Reaktors (1) angeordnetes kreiszylindrisches oder prismatisches, gasdichtes Gehäuse G in
   - einen Innenbereich A, mit einer oder mehreren katalytisch aktiven Zonen (5), worin jeweils eine Packung aus aufeinander, nebeneinander und hintereinander gestapelten Monolithen (4) und vor jeder katalytisch aktiven Zone (5) jeweils eine Mischzone (6) mit festen Einbauten vorgesehen ist und
   - einen koaxial zum Innenbereich A angeordneten Außenbereich B aufgeteilt ist, und wobei
   - an einem Reaktorende im Anschluss an das Gehäuse G ein Wärmetauscher (12) vorgesehen ist
   - mit einer oder mehreren Zuführleitungen (7) für den zu dehydrierenden kohlenwasserstoffhaltigen Gasstrom (2),
   - mit einer oder mehreren, unabhängig voneinander regelbaren Zuführleitungen (9), wobei jede Zuführleitung (9) eine oder mehrere Verteilerkammern (10) versorgt, für den Sauerstoff enthaltenden Gasstrom (3) in jede der Mischzonen (6) sowie
   - mit einer Abführleitung (11) für das Reaktionsgasgemisch der autothermen Gasphasendehydrierung,
   **dadurch gekennzeichnet, dass**
   - der Außenbereich B mit einem unter den Reaktionsbedingungen der autothermen Gasphasendehydrierung inerten Gas beaufschlagbar ist, und dass
   - der zu dehydrierende kohlenwasserstoffhaltige Gasstrom (2) über eine Zuführleitung (7) in den Wärmetauscher (12) eingeleitet, im Wärmetauscher (12) durch das Reaktionsgasgemisch im Gegenstrom durch indirekten Wärmetausch aufgeheizt und weiter an das dem Wärmetauscher (12) entgegengesetzte Ende des Reaktors geleitet, dort umgelenkt, über einen Strömungsgleichrichter (8) in den Innenbereich A eingeleitet, und in den Mischzonen (6) mit dem Sauerstoff enthaltenden Gasstrom (3) vermischt wird, worauf im Innenbereich A des Reaktors (1) die autotherme Gasphasendehydrierung stattfindet, und dass im Innenbereich A zwei oder mehrere katalytisch aktive Zonen (5) mit jeweils einer Packung aus aufeinander, nebeneinander und hintereinander gestapelten Monolithen (4) vorgesehen sind.

2. Reaktor (1) nach Anspruch 1, **dadurch gekennzeichnet, dass** der zu dehydrierende kohlenwasserstoffhaltige Gasstrom (2), an zwei oder mehreren Stellen in den Wärmetauscher (12) eingeleitet wird, bevorzugt als ein Hauptstrom mit höherem Massenstrom und ein oder mehrere Nebenströme mit niedrigerem Massenstrom gegenüber dem Hauptstrom.

3. Reaktor (1) nach Anspruch 1 oder 2, **dadurch gekennzeichnet, dass** zusätzlich zum Wärmetauscher (12) eine oder mehrere Zusatzheizungen für den zu dehydrierenden kohlenwasserstoffhaltigen Gasstrom (2) vorgesehen sind.

4. Reaktor (1) nach Anspruch 3, **dadurch gekennzeichnet, dass** als Zusatzheizung für den kohlenwasserstoffhaltigen Gasstrom (2) die Zuführung von Wasserstoff über eine Leitung (23) in die Zuführleitung (7) für den zu dehydrierenden kohlenwasserstoffhaltigen Gasstrom (2), möglichst nahe am Eintritt in die Mischzonen (6), die vor jeder katalytisch aktiven Zone (5) angeordnet sind, vorgesehen ist.

5. Reaktor (1) nach Anspruch 3 oder 4, **dadurch gekennzeichnet, dass** als Zusatzheizung eine elektrische Heizung (22) vorgesehen ist, die bevorzugt lösbar, als Einstecksystem, innerhalb des Außenbereichs B des Reaktors (1) eingebracht wird, oder als Muffelbrenner (22), in die Zuführleitung (7) für den zu dehydrierenden kohlenwasserstoffhaltigen Gasstrom (2) nach dem Austritt desselben aus dem Wärmetauscher (12).

6. Reaktor (1) nach einem der Ansprüche 1 bis 5, **dadurch gekennzeichnet, dass** die Monolithe (4) innerhalb derselben katalytisch aktiven Zone (5) mit jeweils unterschiedliche katalytischer Aktivität ausgebildet sind.

7. Reaktor (1) nach Anspruch 1 oder 6, **dadurch gekennzeichnet, dass** die zwei oder mehreren katalytisch aktiven Zonen (5) mit jeweils unterschiedlicher katalytischer Aktivität ausgebildet sind.

8. Reaktor (1) nach einem der Ansprüche 1 bis 7, **dadurch gekennzeichnet, dass** die nebeneinander, übereinander und hintereinander zu einer Packung gestapelten Monolithen (4) in einer Blähmatte oder in einem Mineralfaservlies eingehüllt und in eine Einhausung mit Verspanneinrichtung eingesetzt sind.

9. Reaktor (1) nach einem der Ansprüche 1 bis 8, **dadurch gekennzeichnet**, das Gehäuse G als Prisma ausgebildet ist, und dass die Seitenwände des als Prisma ausgebildeten Gehäuses G einzeln abnehmbar ausgebildet sind, dergestalt, dass eine komplette Packung oder einzelne Monolithe (4) einer Packung aus einer katalytisch aktiven Zone (5) ausgetauscht werden können.

10. Reaktor (1) nach einem der Ansprüche 1 bis 9, **dadurch gekennzeichnet, dass** das Gehäuse G aus einem Werkstoff gebildet ist, der hitzebeständig ist, insbesondere aus einem Edelstahl, bevorzugt aus einem Edelstahl mit der Werkstoffnummer 1.4541, 1.4910 oder 1.4841.

11. Reaktor (1) nach einem der Ansprüche 1 bis 10, **dadurch gekennzeichnet, dass** der Mantel des Reaktors (1) aus einem für Druckbehälter zugelassenen unlegierten oder niedrig legierten Stahl gebildet ist, insbesondere aus Schwarzstahl, bevorzugt aus Kesselblech HII, oder einem legierten Stahl mit der Werkstoffnummer 1.4541 oder 1.4910.

12. Reaktor (1) nach einem der Ansprüche 1 bis 11, **dadurch gekennzeichnet, dass** jede Mischzone (6) jeweils einen Rohrverteiler umfasst, gebildet aus einer Vielzahl von parallel zueinander, in einer Ebene senkrecht zur Längsrichtung des Reaktors (1) angeordneten Einsteckrohren (14), die mit einer oder mehreren der Verteilerkammern (10) verbunden sind, und die eine Vielzahl von gleichmäßig zueinander beabstandeten Austrittsöffnungen (15) für den Sauerstoff enthaltenden Gasstrom (3) aus dem Einsteckrohr (14) aufweisen, sowie einer Vielzahl von gleichmäßig zueinander beabstandeten Mischkörpern (16).

13. Reaktor (1) nach Anspruch 12, **dadurch gekennzeichnet, dass** die Mischkörper (16) als Mischplatten ausgebildet sind.

14. Reaktor (1) nach einem der Ansprüche 1 bis 13, **dadurch gekennzeichnet, dass** als Wärmetauscher (12) ein Rohrbündelwärmetauscher eingesetzt wird, der aus einem hoch warmfesten Edelstahl, insbesondere einem Edelstahl mit der Werkstoffnummer 1.4541 oder 1.4910 gebildet ist, dessen Rohre an beiden Enden derselben in Rohrböden spaltfrei durch Hinterbodenschweißung eingebracht sind und dessen Rohrböden auf der Heißgasseite derselben mit einem hitzebeständigen Edelstahl, insbesondere mit einem Edelstahl mit der Werkstoffnummer 1.4841, plattiert sind.

15. Verfahren zur Durchführung einer autothermen Gasphasendehydrierung unter Verwendung eines oder mehrerer Reaktoren (1) nach einem der Ansprüche 1 bis 14.

16. Verfahren nach Anspruch 15, **dadurch gekennzeichnet, dass** das unter den Reaktionsbedingungen der autothermen Gasphasendehydrierung inerte Gas Wasserdampf ist.

17. Verfahren nach Anspruch 15 oder 16, **dadurch gekennzeichnet, dass** das unter den Reaktionsbedingungen der autothermen Gasphasendehydrierung inerte Gas als Purge-Gasstrom mit einem Massenstrom von 1/5 bis 1/100, bevorzugt 1/10 bis 1/50, bezogen auf den Massenstrom des kohlenwasserstoffhaltigen Gasstromes (2) unter geringem Überdruck von 2 bis 50 mbar, bevorzugt von 25 bis 30 mbar, bezogen auf den Druck im Innenbereich A durch den Außenbereich B geleitet wird, bevorzugt, indem der Purge-Gasstrom an einem Reaktorende über eine oder mehrere Zuführleitungen (20) in den Außenbereich B des Reaktors eingeleitet und am entgegengesetzten Reaktorende in den Innenbereich A des Reaktors weitergeleitet wird, insbesondere über eine oder mehrere, vorteilhaft in einem Winkel verschieden von 90° zur Zuführleitung (7) für den zu dehydrierenden kohlenwasserstoffhaltigen Gasstrom (2) angeordnete Verbindungsleitung(en) (21).

18. Verfahren nach einem der Ansprüche 15 bis 17, **dadurch gekennzeichnet, dass** zwei oder mehrere Reaktoren (1) nach einem der Ansprüche 1 bis 17 eingesetzt werden, wobei mindestens ein Reaktor (1) für die autotherme Gasphasendehydrierung genutzt und gleichzeitig mindestens ein weiterer Reaktor (1) regeneriert wird.

19. Verfahren nach Anspruch 18, **dadurch gekennzeichnet, dass** die Regenerierung in einem Temperaturbereich zwischen 550 und 700°C durchgeführt wird.

**20.** Verfahren nach Anspruch 18 oder 19, **dadurch gekennzeichnet, dass** die Regenerierung mit einem sauerstoffhaltigen Gasstrom, enthaltend 0,1 bis 1,5 Gew.-% Sauerstoff, bezogen auf das Gesamtgewicht des sauerstoffhaltigen Gasstromes, durchgeführt wird.

**21.** Verfahren nach einem der Ansprüche 15 bis 20, **dadurch gekennzeichnet, dass** die autotherme Gasphasendehydrierung eine Dehydrierung von Propan, von Butan, von Isobutan, von Buten zu Butadien oder von Ethylbenzol zu Styrol ist.

**22.** Verfahren nach Anspruch 21, **dadurch gekennzeichnet, dass** die autotherme Gasphasendehydrierung die Dehydrierung von Butan ist, und dass zwei Reaktoren (1) eingesetzt werden, wobei ein Reaktor (1) für die autotherme Gasphasendehydrierung genutzt und gleichzeitig ein zweiter Reaktor (1) regeneriert wird.

**23.** Verfahren nach Anspruch 22, **dadurch gekennzeichnet, dass** die Regenerierung die folgenden Verfahrensschritte umfasst:

- Inertisieren des Reaktors (1),
- Abbrennen der Koksablagerungen an der Oberfläche des heterogenen Katalysators,
- Redispergieren des heterogenen Katalysators mit Luft,
- Spülen des heterogenen Katalysators und
- Reduzieren des heterogenen Katalysators.

**Claims**

**1.** A reactor (1) in the form of a horizontal cylinder or prism for carrying out an autothermal gas-phase dehydrogenation of a hydrocarbon-comprising gas stream (2) by means of an oxygen-comprising gas stream (3) to give a reaction gas mixture over a heterogeneous catalyst configured as monolith (4), where

- the interior of the reactor (1) is divided by a detachable, cylindrical or prismatic, gastight housing G which is arranged in the longitudinal direction of the reactor (1) into
- an inner region A having one or more catalytically active zones (5), in which a packing composed of monoliths (4) stacked on top of one another, next to one another and behind one another and before each catalytically active zone (5) in each case a mixing zone (6) having solid internals are provided, and
- an outer region B arranged coaxially to the inner region A, and
- a heat exchanger (12) is provided at one end of the reactor connected to the housing G,
- with one or more feed lines (7) for the hydrocarbon-comprising gas stream to be dehydrogenated (2),
- with one or more feed lines (9) which can be regulated independently of one another, where each feed line (9) supplies one or more distribution chambers (10), for the oxygen-comprising gas stream (3) into each of the mixing zones (6) and
- with a discharge line (11) for the reaction gas mixture of the autothermal gas-phase dehydrogenation, wherein
- the outer region B can be supplied with a gas which is inert under the reaction conditions of the autothermal gas-phase dehydrogenation and
- the hydrocarbon-comprising gas stream (2) to be dehydrogenated is introduced via a feed line (7) into the heat exchanger (12), is heated in the heat exchanger (12) by means of the reaction gas mixture in countercurrent by indirect heat exchange and conveyed further to the end of the reactor opposite the heat exchanger (12), redirected there, introduced via a flow conditioner (8) into the inner region A and mixed with the oxygen-comprising gas stream (3) in the mixing zones (6), whereupon the autothermal gas-phase dehydrogenation takes place in the inner region A of the reactor (1), and two or more catalytically active zones (5) each having a packing composed of monoliths (4) stacked on top of, next to and behind one another are provided in the inner region A.

**2.** The reactor (1) according to claim 1, wherein the hydrocarbon-comprising gas stream (2) to be hydrogenated is introduced into the heat exchanger (12) at two or more places, preferably as a main stream having a higher mass flow and one or more secondary streams having a lower mass flow compared to the main stream.

**3.** The reactor (1) according to claim 1 or 2, wherein one or more supplementary heating devices for the hydrocarbon-comprising gas stream (2) to be dehydrogenated are provided in addition to the heat exchanger (12).

4.  The reactor (1) according to claim 3, wherein introduction of hydrogen via a line (23) into the feed line (7) for the hydrocarbon-comprising gas stream (2) to be dehydrogenated, as close as possible to the inlet into the mixing zones (6) which are arranged upstream of each catalytically active zone (5), is provided as supplementary heating for the hydrocarbon-comprising gas stream (2).

5.  The reactor (1) according to claim 3 or 4, wherein electric heating (22) which is preferably installed detachably, as plug-in system, within the outer region B of the reactor (1) or as muffle burner (22) into the feed line (7) for the hydrocarbon-comprising gas stream (2) to be dehydrogenated after the latter has exited from the heat exchanger (12) is provided as supplementary heating.

6.  The reactor (1) according to any of claims 1 to 5, wherein the monoliths (4) within the same catalytically active zone (5) have in each case a different catalytic activity.

7.  The reactor (1) according to claim 1 or 6, wherein the two or more catalytically active zones (5) have in each case a different catalytic activity.

8.  The reactor (1) according to any of claims 1 to 7, wherein the monoliths (4) which are stacked next to, above and behind one another to form a packing are enveloped in an expandable mat or a mineral fiber nonwoven and installed in a housing with a tensioning device.

9.  The reactor (1) according to any of claims 1 to 8, wherein the housing G is configured as a prism, and the side walls of the housing G configured as a prism can be taken off individually so that a complete packing or individual monoliths (4) of a packing from a catalytically active zone (5) can be replaced.

10. The reactor (1) according to any of claims 1 to 9, wherein the housing G is made of a material which is heat-resistant, in particular a stainless steel, preferably a stainless steel having the material number 1.4541, 1.4910 or 1.4841.

11. The reactor (1) according to any of claims 1 to 10, wherein the outer wall of the reactor (1) is made of a nonalloy or low-alloy steel approved for pressure vessels, in particular black steel, preferably boiler plate HII, or an alloy steel having the material number 1.4541 or 1.4910.

12. The reactor (1) according to any of claims 1 to 11, wherein each mixing zone (6) comprises a tube manifold formed by a plurality of parallel plug-in tubes (14) which are arranged in a plane perpendicular to the longitudinal direction of the reactor (1) and are connected to one or more of the distributor chambers (10) and have a plurality of uniformly spaced outlet openings (15) for the oxygen-comprising gas stream (3) from the plug-in tube (14) and also a plurality of uniformly spaced mixing elements (16).

13. The reactor (1) according to claim 12, wherein the mixing elements (16) are configured as mixing plates.

14. The reactor (1) according to any of claims 1 to 13, wherein a shell-and-tube heat exchanger which is made of a highly heat-resistant stainless steel, in particular a stainless steel having the material number 1.4541 or 1.4910, and whose tubes are installed at both ends of the tubes in tube plates without leaving a gap by backplate welding and whose tube plates are clad on the hot gas side of the heat exchanger with a heat-resistant stainless steel, in particular a stainless steel having the material number 1.4841 is used as heat exchanger (12).

15. A process for carrying out an autothermal gas-phase dehydrogenation using one or more reactors (1) according to any of claims 1 to 14.

16. The process according to claim 15, wherein the gas which is inert under the reaction conditions of the autothermal gas-phase dehydrogenation is steam.

17. The process according to claim 15 or 16, wherein the gas which is inert under the reaction conditions of the autothermal gas-phase dehydrogenation is passed as a purge gas stream at a mass flow of from 1/5 to 1/100, preferably from 1/10 to 1/50, based on the mass flow of the hydrocarbon-comprising gas stream (2) under a slight gauge pressure of from 2 to 50 mbar, preferably from 25 to 30 mbar, based on the pressure in the inner region A, through the outer region B, preferably by introducing the purge gas stream via one or more feed lines (20) into the outer region B of the reactor at one end of the reactor and conveying it further into the inner region A of the reactor at the opposite end of the reactor, in particular via one or more connecting line(s) (21) which are advantageously arranged at an angle other than 90° to the feed line (7) for the hydrocarbon-comprising gas stream (2) to be dehydrogenated.

18. The process according to any of claims 15 to 17, wherein two or more reactors (1) according to any of claims 1 to 17 are used, with at least one reactor (1) being utilized for the autothermal gas-phase dehydrogenation and at least one further reactor (1) being regenerated at the same time.

19. The process according to claim 18, wherein the regeneration is carried out in a temperature range from 550 to 700°C.

20. The process according to claim 18 or 19, wherein the regeneration is carried out using an oxygen-comprising gas stream comprising from 0.1 to 1.5% by weight of oxygen, based on the total weight of the oxygen-comprising gas stream.

21. The process according to any of claims 15 to 20, wherein the autothermal gas-phase dehydrogenation is a dehydrogenation of propane, of butane, of isobutane, of butene to butadiene or of ethylbenzene to styrene.

22. The process according to claim 21, wherein the autothermal gas-phase dehydrogenation is the dehydrogenation of butane and two reactors (1) are used, with one reactor (1) being utilized for the autothermal gas-phase dehydrogenation and a second reactor (1) being regenerated at the same time.

23. The process according to claim 22, wherein the regeneration comprises the following process steps:

   - making the reactor (1) inert,
   - burning-off of the carbonaceous deposits on the surface of the heterogeneous catalyst,
   - redispersion of the heterogeneous catalyst by means of air,
   - flushing of the heterogeneous catalyst and
   - reduction of the heterogeneous catalyst.

**Revendications**

1. Réacteur (1) sous forme d'un cylindre ou prisme horizontal, destiné à la mise en oeuvre d'une déshydrogénation en phase gazeuse autothermique d'un courant de gaz (2) contenant un hydrocarbure à l'aide d'un courant de gaz (3) contenant de l'oxygène, avec obtention d'un mélange de gaz de réaction, sur un catalyseur hétérogène qui se trouve sont forme de monolithe (4), dans lequel

   - l'intérieur du réacteur (1) est divisé par une enceinte G étanche aux gaz, amovible, cylindrique circulaire ou prismatique disposée dans le sens de la longueur du réacteur (1), en
   - une partie interne A, comportant une ou plusieurs zones catalytiquement actives (5), dans lesquelles est prévu chaque fois un empilement de monolithes (4) tassés les uns sur les autres, les uns à côté des autres et les uns derrière les autres et devant chaque zone catalytiquement active (5) est chaque fois prévue une zone de mélange (6) à inserts fixes et
   - une partie externe B disposée coaxialement avec la partie interne A, et dans lequel
   - à une extrémité du réacteur est prévu à la suite de l'enceinte G un échangeur thermique (12)
   - avec un ou plusieurs conduits d'arrivée (7) pour le courant de gaz (2) contenant un hydrocarbure à déshydrogéner,
   - avec un ou plusieurs conduits d'alimentation (9) réglables indépendamment les uns des autres, chaque conduit d'alimentation (9) alimentant une ou plusieurs chambres de distribution (10) pour le courant de gaz (3) contenant de l'oxygène, dans chacune des zones de mélange (6) ainsi
   - qu'avec un conduit d'évacuation (11) pour le mélange de gaz de réaction de la déshydrogénation en phase gazeuse autothermique,
   **caractérisé en ce que**
   - la partie externe B peut être alimentée en un gaz inerte dans les conditions réactionnelles de la déshydrogénation en phase gazeuse autothermique, et **en ce que**
   - le courant de gaz (2) contenant un hydrocarbure à déshydrogéner est introduit par un conduit d'alimentation (7) dans l'échangeur thermique (12), chauffé à contre-courant par échange thermique indirect dans l'échangeur thermique (12) par le mélange de gaz de réaction et envoyé ultérieurement à l'extrémité opposée à l'échangeur thermique (12) du réacteur, y est dévié, introduit via un redresseur de flux (8) dans la partie interne A, et est mélangé dans les zones de mélange (6) avec le courant de gaz (3) contenant de l'oxygène, à la suite de quoi la déshydrogénation en phase gazeuse autothermique a lieu dans la partie interne A du réacteur (1), et **en ce que** dans la partie interne A sont prévues deux ou plus de deux zones catalytiquement actives (5) comportant chacune un empilement de monolithes (4) tassés les uns sur les autres, les uns à côté des autres et les uns derrière les autres.

2.  Réacteur (1) selon la revendication 1, **caractérisé en ce que** le courant de gaz (2) contenant un hydrocarbure à déshydrogéner est envoyé en deux ou plusieurs points dans l'échangeur thermique (12), de préférence sous forme d'un courant principal à débit massique plus fort et d'un ou de plusieurs courants secondaires à débit massique plus faible par rapport au courant principal.

3.  Réacteur (1) selon la revendication 1 ou 2, **caractérisé en ce qu'**en plus de l'échangeur thermique (12) sont prévus un ou plusieurs chauffages supplémentaires pour le courant de gaz (2) contenant un hydrocarbure à déshydrogéner.

4.  Réacteur (1) selon la revendication 3, **caractérisé en ce qu'**en tant que chauffage supplémentaire pour le courant de gaz (2) contenant un hydrocarbure est prévue l'introduction d'hydrogène par un conduit (23) dans le conduit d'alimentation (7) pour le courant de gaz (2) contenant un hydrocarbure à déshydrogéner, le plus près possible de l'entrée dans les zones de mélange (6) qui sont disposées devant chaque zone catalytiquement active (5).

5.  Réacteur (1) selon la revendication 3 ou 4, **caractérisé en ce qu'**en tant que chauffage supplémentaire est prévu un chauffage électrique (22) qui est introduit, de préférence de façon amovible, sous forme de système enfichable, à l'intérieur de la partie externe B du réacteur (1), ou sous forme de brûleur à moufle (22), dans le conduit d'alimentation (7) pour le courant de gaz (2) contenant un hydrocarbure à déshydrogéner, après la sortie de celui-ci de l'échangeur thermique (12).

6.  Réacteur (1) selon l'une quelconque des revendications 1 à 5, **caractérisé en ce qu'**à l'intérieur de la même zone catalytiquement active (5) les monolithes (4) sont conçus pour avoir chacun une activité catalytique différente.

7.  Réacteur (1) selon la revendication 1 ou 6, **caractérisé en ce que** les deux ou plus de deux zones catalytiquement actives (5) sont conçues pour avoir chacune une activité catalytique différente.

8.  Réacteur (1) selon l'une quelconque des revendications 1 à 7, **caractérisé en ce que** les monolithes (4) tassés en un empilement les uns à côté des autres, les uns sur les autres et les uns derrière les autres sont enveloppés dans un capitonnage ou dans un non-tissé en fibres minérales et insérés dans un boîtier avec dispositif de serrage.

9.  Réacteur (1) selon l'une quelconque des revendications 1 à 8, **caractérisé en ce que** l'enceinte G est en forme de prisme, et **en ce que** les parois latérales de l'enceinte G en forme de prisme sont individuellement amovibles, de sorte qu'un empilement complet ou des monolithes (4) individuels d'un empilement d'une zone catalytiquement active (5) peuvent être remplacés.

10. Réacteur (1) selon l'une quelconque des revendications 1 à 9, **caractérisé en ce que** l'enceinte G est constituée d'un matériau qui est résistant à la chaleur, en particulier d'un acier spécial, de préférence d'un acier spécial portant le numéro de matériau 1.4541, 1.4910 ou 1.4841.

11. Réacteur (1) selon l'une quelconque des revendications 1 à 10, **caractérisé en ce que** l'enveloppe du réacteur (1) est constituée d'un acier non allié ou faiblement allié admissible pour des récipients tenant la pression, en particulier d'acier noir, de préférence de tôle pour chaudières HII, ou d'un acier allié portant le numéro de matériau 1.4541 ou 1.4910.

12. Réacteur (1) selon l'une quelconque des revendications 1 à 11, **caractérisé en ce que** chaque zone de mélange (6) comprend chaque fois un manifold, constitué d'une multiplicité de tubes enfichables (14) disposés parallèlement les uns aux autres, dans un plan perpendiculaire au sens longitudinal du réacteur (1), qui sont reliés à une ou plusieurs des chambres de distribution (10), et qui comportent une multiplicité d'orifices de sortie (15) régulièrement espacés les uns des autres pour le courant de gaz (3) contenant de l'oxygène, à partir du tube enfichable (14), ainsi que d'une multiplicité de corps de mélange (16) espacés régulièrement les uns des autres.

13. Réacteur (1) selon la revendication 12, **caractérisé en ce que** les corps de mélange (16) sont en forme de plaques de mélange.

**14.** Réacteur (1) selon l'une quelconque des revendications 1 à 13, **caractérisé en ce qu'**on utilise comme échangeur thermique (12) un échangeur thermique à faisceau de tubes qui est constitué d'un acier spécial hautement résistant à la chaleur, en particulier d'un acier spécial portant le numéro de matériau 1.4541 ou 1.4910, dont les tubes sont insérés par soudage par le fond, sans interstice, à leurs deux extrémités, dans des plaques de support de tubes et dont les plaques de support de tubes sont plaquées du côté gaz chaud avec un acier spécial résistant à la chaleur, en particulier avec un acier spécial portant le numéro de matériau 1.4841.

**15.** Procédé pour la mise en oeuvre d'une déshydrogénation en phase gazeuse autothermique avec utilisation d'un ou de plusieurs réacteurs (1) selon l'une quelconque des revendications 1 à 14.

**16.** Procédé selon la revendication 15, **caractérisé en ce que** le gaz inerte dans les conditions réactionnelles de la déshydrogénation en phase gazeuse autothermique est de la vapeur d'eau.

**17.** Procédé selon la revendication 15 ou 16, **caractérisé en ce qu'**on fait passer dans la partie externe B le gaz inerte dans les conditions réactionnelles de la déshydrogénation en phase gazeuse autothermique, en tant que courant de gaz de purge, à un débit massique de 1/5 à 1/100, de préférence de 1/10 à 1/50, par rapport au débit massique du courant de gaz (2) contenant un hydrocarbure, sous une faible surpression de 2 à 50 mbars, de préférence de 25 à 30 mbars, par rapport à la pression dans la partie interne A, en introduisant le courant de gaz de purge à une extrémité du réacteur par un ou plusieurs conduits d'alimentation (20) et à l'extrémité opposée du réacteur on le fait encore passer dans la partie interne A du réacteur, en particulier par un ou plusieurs conduit(s) de raccordement (21) disposés avantageusement en faisant un angle différent de 90° avec le conduit d'alimentation (7) pour le courant de gaz (2) contenant un hydrocarbure à déshydrogéner.

**18.** Procédé selon l'une quelconque des revendications 15 à 17, **caractérisé en ce qu'**on utilise deux ou plus de deux réacteurs (1) selon l'une quelconque des revendications 1 à 17, au moins un réacteur (1) étant utilisé pour la déshydrogénation en phase gazeuse autothermique et en même temps au moins un autre réacteur (1) étant régénéré.

**19.** Procédé selon la revendication 18, **caractérisé en ce que** la régénération est effectuée dans une plage de température comprise entre 550 et 700 °C.

**20.** Procédé selon la revendication 18 ou 19, **caractérisé en ce qu'**on effectue la régénération avec un courant de gaz contenant de l'oxygène, contenant 0,1 à 1,5 % en poids d'oxygène, par rapport au poids total du courant de gaz contenant de l'oxygène.

**21.** Procédé selon l'une quelconque des revendications 15 à 20, **caractérisé en ce que** la déshydrogénation en phase gazeuse autothermique est une déshydrogénation de propane, de butane, d'isobutane, de butène en butadiène ou d'éthylbenzène en styrène.

**22.** Procédé selon la revendication 21, **caractérisé en ce que** la déshydrogénation en phase gazeuse autothermique est la déshydrogénation de butane, et **en ce qu'**on utilise deux réacteurs (1), un réacteur (1) étant utilisé pour la déshydrogénation en phase gazeuse autothermique et en même temps un deuxième réacteur (1) étant régénéré.

**23.** Procédé selon la revendication 22, **caractérisé en ce que** la régénération comprend les étapes de processus suivantes :

   - inertisation du réacteur (1),
   - séparation des dépôts de coke à la surface du catalyseur hétérogène,
   - redispersion du catalyseur hétérogène avec de l'air,
   - lavage du catalyseur hétérogène et
   - réduction du catalyseur hétérogène.

FIG.1

FIG.2

## FIG.3

Section B-B

## FIG.4

Section A-A

# FIG.5

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 7034195 B
- US 20080119673 A
- EP 09177649 A
- DE 4026566 A